(19)

Europäisches
Patentamt
European
Patent Office
Office européen
des brevets

(11)    EP 2 197 918 B1

(12)        **EUROPEAN PATENT SPECIFICATION**

(45) Date of publication and mention
of the grant of the patent:
**18.12.2013   Bulletin 2013/51**

(51) Int Cl.:
*C07K 16/28* (2006.01)        *A61K 39/395* (2006.01)
*A61P 35/02* (2006.01)

(21) Application number: **08785642.3**

(22) Date of filing: **20.08.2008**

(86) International application number:
**PCT/EP2008/006833**

(87) International publication number:
**WO 2009/030368 (12.03.2009 Gazette 2009/11)**

(54) **COMBINATION THERAPY WITH TYPE I AND TYPE II ANTI-CD20 ANTIBODIES**

KOMBINATIONSTHERAPIE MIT TYP-I- UND TYP-II-ANTI-CD20-ANTIKÖRPERN

POLYTHÉRAPIE AVEC DES ANTICORPS ANTI-CD20 DE TYPE I ET DE TYPE II

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **05.09.2007   EP 07017337**

(43) Date of publication of application:
**23.06.2010   Bulletin 2010/25**

(73) Proprietors:
• **F. Hoffmann-La Roche AG**
**4070 Basel (CH)**
• **Roche Glycart AG**
**8952 Schlieren-Zuerich (CH)**

(72) Inventors:
• **FRIESS, Thomas**
**86911 Diessen-Dettenhofen (DE)**
• **KLEIN, Christian**
**8906 Bonstetten (CH)**
• **UMANA, Pablo**
**8820 Waedenswil (CH)**

(74) Representative: **Vossius & Partner**
**P.O. Box 86 07 67**
**81634 München (DE)**

(56) References cited:
**WO-A-2004/035607     WO-A-2007/031875**

• **GLENNIE ET AL: "Mechanisms of killing by anti-CD20 monoclonal antibodies" MOLECULAR IMMUNOLOGY, ELMSFORD, NY, US, vol. 44, no. 16, 4 September 2007 (2007-09-04), pages 3823-3837, XP022227562 ISSN: 0161-5890 Retrieved from the Internet: URL:http://www.sciencedirect.com/science?_ob=ArticleURL&_udi=B6T9R-4PK8TMS-4&_user=9 87766&_rdoc=1&_fmt=&_orig=search&_sort=d&v iew=c&_acct=C000049880&_version=1&_urlVers ion=0&_userid=987766&md5=a7936d5ec69cc7a3b f63e7c48e9efb40> [retrieved on 2007-01-17]**
• **CRAGG MARK S ET AL: "Antibody specificity controls in vivo effector mechanisms of anti-CD20 reagents" BLOOD, vol. 103, no. 7, 1 April 2004 (2004-04-01), pages 2738-2743, XP002465311 ISSN: 0006-4971**
• **CRAGG M S ET AL: "Complement-mediated lysis by anti-CD20 mAb correlates with segregation into lipid rafts" BLOOD, W.B.SAUNDERS COMPANY, ORLANDO, FL, US, vol. 101, no. 3, 1 February 2003 (2003-02-01), pages 1045-1052, XP002329935 ISSN: 0006-4971**
• **CARDARELLI PINA M ET AL: "Binding to CD20 by Anti-B1 Antibody or F(ab')2 is sufficient for induction of apoptosis in B-cell lines" CANCER IMMUNOLOGY AND IMMUNOTHERAPY, BERLIN, DE, vol. 51, no. 1, March 2002 (2002-03), pages 15-24, XP002308043 ISSN: 0340-7004**

**EP 2 197 918 B1**

SEQUENCE LISTING

<110> F. Hoffmann-La Roche AG

<120> Combination therapy with type I and type II anti-CD20 antibodies

<130> 24509

<150> EP 07017337
<151> 2007-09-05

<160> 20

<170> PatentIn version 3.2

<210> 1
<211> 112
<212> PRT
<213> Mus sp.

<220>
<221> MISC_FEATURE
<223> amino acid sequence of variable region of the heavy chain (VH) of murine monoclonal anti-CD20 antibody B-Ly1

<400> 1

```
Gly Pro Glu Leu Val Lys Pro Gly Ala Ser Val Lys Ile Ser Cys Lys
1               5                   10                  15

Ala Ser Gly Tyr Ala Phe Ser Tyr Ser Trp Met Asn Trp Val Lys Leu
            20                  25                  30

Arg Pro Gly Gln Gly Leu Glu Trp Ile Gly Arg Ile Phe Pro Gly Asp
        35                  40                  45

Gly Asp Thr Asp Tyr Asn Gly Lys Phe Lys Gly Lys Ala Thr Leu Thr
    50                  55                  60

Ala Asp Lys Ser Ser Asn Thr Ala Tyr Met Gln Leu Thr Ser Leu Thr
65                  70                  75                  80

Ser Val Asp Ser Ala Val Tyr Leu Cys Ala Arg Asn Val Phe Asp Gly
            85                  90                  95

Tyr Trp Leu Val Tyr Trp Gly Gln Gly Thr Leu Val Thr Val Ser Ala
            100                 105                 110
```

<210> 2
<211> 103
<212> PRT
<213> Mus sp.

<220>
<221> MISC_FEATURE
<223> amino acid sequence of variable region of the light chain (VL) of murine monoclonal anti-CD20 antibody B-Ly1

<400> 2

```
        Asn Pro Val Thr Leu Gly Thr Ser Ala Ser Ile Ser Cys Arg Ser Ser
        1               5               10              15

        Lys Ser Leu Leu His Ser Asn Gly Ile Thr Tyr Leu Tyr Trp Tyr Leu
                    20              25              30

        Gln Lys Pro Gly Gln Ser Pro Gln Leu Leu Ile Tyr Gln Met Ser Asn
                35              40              45

        Leu Val Ser Gly Val Pro Asp Arg Phe Ser Ser Ser Gly Ser Gly Thr
            50              55              60

        Asp Phe Thr Leu Arg Ile Ser Arg Val Glu Ala Glu Asp Val Gly Val
        65              70              75              80

        Tyr Tyr Cys Ala Gln Asn Leu Glu Leu Pro Tyr Thr Phe Gly Gly Gly
                    .   85              90              95

        Thr Lys Leu Glu Ile Lys Arg
                    100
```

<210> 3
<211> 119
<212> PRT
<213> Artificial

<220>
<223> amino acid sequences of variable region of the heavy chain (VH) of humanized B-Ly1 antibody (B-HH2)

<400> 3

```
        Gln Val Gln Leu Val Gln Ser Gly Ala Glu Val Lys Lys Pro Gly Ser
        1               5               10              15

        Ser Val Lys Val Ser Cys Lys Ala Ser Gly Tyr Ala Phe Ser Tyr Ser
                    20              25              `   30

        Trp Met Asn Trp Val Arg Gln Ala Pro Gly Gln Gly Leu Glu Trp Met
                    35              40              45
```

```
Gly Arg Ile Phe Pro Gly Asp Gly Asp Thr Asp Tyr Asn Gly Lys Phe
    50              55              60

Lys Gly Arg Val Thr Ile Thr Ala Asp Lys Ser Thr Ser Thr Ala Tyr
65              70              75              80

Met Glu Leu Ser Ser Leu Arg Ser Glu Asp Thr Ala Val Tyr Tyr Cys
            85              90              95

Ala Arg Asn Val Phe Asp Gly Tyr Trp Leu Val Tyr Trp Gly Gln Gly
        100             105             110

Thr Leu Val Thr Val Ser Ser
        115
```

<210> 4
<211> 119
<212> PRT
<213> Artificial

<220>
<223> amino acid sequences of variable region of the heavy chain (VH) of humanized B-Ly1 antibody (B-HH3)

<400> 4

```
Gln Val Gln Leu Val Gln Ser Gly Ala Glu Val Lys Lys Pro Gly Ser
1               5               10              15

Ser Val Lys Val Ser Cys Lys Ala Ser Gly Tyr Ala Phe Ser Tyr Ser
        20              25              30

Trp Met Asn Trp Val Arg Gln Ala Pro Gly Gln Gly Leu Glu Trp Met
        35              40              45

Gly Arg Ile Phe Pro Gly Asp Gly Asp Thr Asp Tyr Asn Gly Lys Phe
    50              55              60

Lys Gly Arg Val Thr Ile Thr Ala Asp Lys Ser Thr Ser Thr Ala Tyr
65              70              75              80

Met Glu Leu Ser Ser Leu Arg Ser Glu Asp Thr Ala Val Tyr Leu Cys
            85              90              95

Ala Arg Asn Val Phe Asp Gly Tyr Trp Leu Val Tyr Trp Gly Gln Gly
        100             105             110

Thr Leu Val Thr Val Ser Ser
        115
```

<210> 5
<211> 119
<212> PRT
<213> Artificial

<220>
<223> amino acid sequences of variable region of the heavy chain (VH) of humanized B-Ly1 antibody (B-HH4)

<400> 5

```
Gln Val Gln Leu Val Gln Ser Gly Ala Glu Val Lys Lys Pro Gly Ala
1               5                   10                  15

Ser Val Lys Val Ser Cys Lys Val Ser Gly Tyr Ala Phe Ser Tyr Ser
            20                  25                  30

Trp Met Asn Trp Val Arg Gln Ala Pro Gly Gln Gly Leu Glu Trp Met
            35                  40                  45

Gly Arg Ile Phe Pro Gly Asp Gly Asp Thr Asp Tyr Asn Gly Lys Phe
        50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asn Val Phe Asp Gly Tyr Trp Leu Val Tyr Trp Gly Gln Gly
            100                 105                 110

Thr Leu Val Thr Val Ser Ser
            115
```

<210> 6
<211> 119
<212> PRT
<213> Artificial

<220>
<223> amino acid sequences of variable region of the heavy chain (VH) of humanized B-Ly1 antibody (B-HH5)

<400> 6

Gln Val Gln Leu Val Gln Ser Gly Ala Glu Val Lys Lys Pro Gly Ser
1               5               10              15

Ser Val Lys Val Ser Cys Lys Ala Ser Gly Tyr Ala Phe Ser Tyr Ser
            20              25              30

Trp Met Ser Trp Val Arg Gln Ala Pro Gly Gln Gly Leu Glu Trp Met
        35              40              45

Gly Arg Ile Phe Pro Gly Asp Gly Asp Thr Asp Tyr Asn Gly Lys Phe
    50              55              60

Lys Gly Arg Val Thr Ile Thr Ala Asp Lys Ser Thr Ser Thr Ala Tyr
65              70              75              80

Met Glu Leu Ser Ser Leu Arg Ser Glu Asp Thr Ala Val Tyr Tyr Cys
            85              90              95

Ala Arg Asn Val Phe Asp Gly Tyr Trp Leu Val Tyr Trp Gly Gln Gly
            100             105             110

Thr Leu Val Thr Val Ser Ser
            115

<210> 7
<211> 119
<212> PRT
<213> Artificial

<220>
<223> amino acid sequences of variable region of the heavy chain (VH) of humanized B-Ly1 antibody (B-HH6)

<400> 7

Gln Val Gln Leu Val Gln Ser Gly Ala Glu Val Lys Lys Pro Gly Ser
1               5               10              15

Ser Val Lys Val Ser Cys Lys Ala Ser Gly Tyr Ala Phe Ser Tyr Ser
            20              25              30

Trp Ile Asn Trp Val Arg Gln Ala Pro Gly Gln Gly Leu Glu Trp Met
        35              40              45

Gly Arg Ile Phe Pro Gly Asp Gly Asp Thr Asp Tyr Asn Gly Lys Phe
    50              55              60

```
Lys Gly Arg Val Thr Ile Thr Ala Asp Lys Ser Thr Ser Thr Ala Tyr
65              70              75                  80

Met Glu Leu Ser Ser Leu Arg Ser Glu Asp Thr Ala Val Tyr Tyr Cys
            85              90                  95

Ala Arg Asn Val Phe Asp Gly Tyr Trp Leu Val Tyr Trp Gly Gln Gly
        100             105             110

Thr Leu Val Thr Val Ser Ser
        115
```

<210> 8
<211> 119
<212> PRT
<213> Artificial

<220>
<223> amino acid sequences of variable region of the heavy chain (VH) of humanized B-Ly1 antibody (B-HH7)

<400> 8

```
Gln Val Gln Leu Val Gln Ser Gly Ala Glu Val Lys Lys Pro Gly Ser
1               5               10                  15

Ser Val Lys Val Ser Cys Lys Ala Ser Gly Tyr Ala Phe Ser Tyr Ser
        20              25                  30

Trp Ile Ser Trp Val Arg Gln Ala Pro Gly Gln Gly Leu Glu Trp Met
        35              40                  45

Gly Arg Ile Phe Pro Gly Asp Gly Asp Thr Asp Tyr Asn Gly Lys Phe
        50              55                  60

Lys Gly Arg Val Thr Ile Thr Ala Asp Lys Ser Thr Ser Thr Ala Tyr
65              70              75                  80

Met Glu Leu Ser Ser Leu Arg Ser Glu Asp Thr Ala Val Tyr Tyr Cys
            85              90                  95

Ala Arg Asn Val Phe Asp Gly Tyr Trp Leu Val Tyr Trp Gly Gln Gly
        100             105             110

Thr Leu Val Thr Val Ser Ser
        115
```

<210> 9
<211> 119
<212> PRT

<210> Artificial

<220>
<223> amino acid sequences of variable region of the heavy chain (VH) of humanized B-Ly1 antibody (B-HH8)

<400> 9

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Tyr Ser
            20                  25                  30

Trp Met Asn Trp Val Arg Gln Ala Pro Gly Gln Gly Leu Glu Trp Met
        35                  40                  45

Gly Arg Ile Phe Pro Gly Asp Gly Asp Thr Asp Tyr Asn Gly Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
            85                  90                  95

Ala Arg Asn Val Phe Asp Gly Tyr Trp Leu Val Tyr Trp Gly Gln Gly
            100                 105                 110

Thr Leu Val Thr Val Ser Ser
            115

<210> 10
<211> 119
<212> PRT
<213> Artificial

<220>
<223> amino acid sequences of variable region of the heavy chain (VH) of humanized B-Ly1 antibody (B-HH9)

<400> 10

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ser Tyr Ser
20 25 30

Trp Met Asn Trp Val Arg Gln Ala Pro Gly Gln Gly Leu Glu Trp Met
35 40 45

Gly Arg Ile Phe Pro Gly Asp Gly Asp Thr Asp Tyr Asn Gly Lys Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asn Val Phe Asp Gly Tyr Trp Leu Val Tyr Trp Gly Gln Gly
100 105 110

Thr Leu Val Thr Val Ser Ser
115

<210> 11
<211> 119
<212> PRT
<213> Artificial

<220>
<223> amino acid sequences of variable region of the heavy chain (VH) of humanized B-Ly1 antibody (B-HL8)

<400> 11

Glu Val Gln Leu Val Glu Ser Gly Gly Gly Leu Val Lys Pro Gly Gly
1 5 10 15

Ser Leu Arg Leu Ser Cys Ala Ala Ser Gly Phe Thr Phe Ser Tyr Ser
20 25 30

Trp Met Asn Trp Val Arg Gln Ala Pro Gly Lys Gly Leu Glu Trp Val
35 40 45

Gly Arg Ile Phe Pro Gly Asp Gly Asp Thr Asp Tyr Asn Gly Lys Phe
50 55 60

Lys Gly Arg Val Thr Ile Thr Ala Asp Lys Ser Thr Ser Thr Ala Tyr
65 70 75 80

```
    Met Glu Leu Ser Ser Leu Arg Ser Glu Asp Thr Ala Val Tyr Tyr Cys
                    85                  90                  95

    Ala Arg Asn Val Phe Asp Gly Tyr Trp Leu Val Tyr Trp Gly Gln Gly
                    100                 105                 110

    Thr Leu Val Thr Val Ser Ser
                    115
```

<210> 12
<211> 119
<212> PRT
<213> Artificial

<220>
<223> amino acid sequences of variable region of the heavy chain (VH) of humanized B-Ly1 antibody (B-HL10)

<400> 12

```
    Glu Val Gln Leu Val Glu Ser Gly Gly Gly Leu Val Lys Pro Gly Gly
    1               5                   10                  15

    Ser Leu Arg Leu Ser Cys Ala Ala Ser Gly Phe Ala Phe Ser Tyr Ser
                    20                  25                  30

    Trp Met Asn Trp Val Arg Gln Ala Pro Gly Lys Gly Leu Glu Trp Val
                    35                  40                  45

    Gly Arg Ile Phe Pro Gly Asp Gly Asp Thr Asp Tyr Asn Gly Lys Phe
            50                  55                  60

    Lys Gly Arg Val Thr Ile Thr Ala Asp Lys Ser Thr Ser Thr Ala Tyr
    65                  70                  75                  80

    Met Glu Leu Ser Ser Leu Arg Ser Glu Asp Thr Ala Val Tyr Tyr Cys
                    85                  90                  95

    Ala Arg Asn Val Phe Asp Gly Tyr Trp Leu Val Tyr Trp Gly Gln Gly
                    100                 105                 110

    Thr Leu Val Thr Val Ser Ser
                    115
```

<210> 13
<211> 119
<212> PRT
<213> Artificial

<220>
<223> amino acid sequences of variable region of the heavy chain (VH) of humanized B-Ly1 antibody (B-HL11)

<400> 13

Gln Val Gln Leu Val Glu Ser Gly Gly Gly Leu Val Lys Pro Gly Gly
1               5               10              15

Ser Leu Arg Leu Ser Cys Ala Ala Ser Gly Phe Thr Phe Ser Tyr Ser
            20              25              30

Trp Met Asn Trp Val Arg Gln Ala Pro Gly Lys Gly Leu Glu Trp Val
        35              40              45

Gly Arg Ile Phe Pro Gly Asp Gly Asp Thr Asp Tyr Asn Gly Lys Phe
        50              55              60

Lys Gly Arg Val Thr Ile Thr Ala Asp Lys Ser Thr Ser Thr Ala Tyr
65              70              75              80

Met Glu Leu Ser Ser Leu Arg Ser Glu Asp Thr Ala Val Tyr Tyr Cys
            85              90              95

Ala Arg Asn Val Phe Asp Gly Tyr Trp Leu Val Tyr Trp Gly Gln Gly
            100             105             110

Thr Leu Val Thr Val Ser Ser
            115

<210> 14
<211> 119
<212> PRT
<213> Artificial

<220>
<223> amino acid sequences of variable region of the heavy chain (VH) of humanized B-Ly1 antibody (B-HL12)

<400> 14

Glu Val Gln Leu Val Glu Ser Gly Ala Gly Leu Val Lys Pro Gly Gly
1               5               10              15

Ser Leu Arg Leu Ser Cys Ala Ala Ser Gly Phe Thr Phe Ser Tyr Ser
            20              25              30

Trp Met Asn Trp Val Arg Gln Ala Pro Gly Lys Gly Leu Glu Trp Met

|  | 35 | | | | 40 | | | | 45 | | | |
|---|---|---|---|---|---|---|---|---|---|---|---|---|

Gly Arg Ile Phe Pro Gly Asp Gly Asp Thr Asp Tyr Asn Gly Lys Phe
    50              55              60

Lys Gly Arg Val Thr Ile Thr Ala Asp Lys Ser Thr Ser Thr Ala Tyr
65              70              75              80

Met Glu Leu Ser Ser Leu Arg Ser Glu Asp Thr Ala Val Tyr Tyr Cys
                85              90              95

Ala Arg Asn Val Phe Asp Gly Tyr Trp Leu Val Tyr Trp Gly Gln Gly
            100             105             110

Thr Leu Val Thr Val Ser Ser
            115

<210> 15
<211> 119
<212> PRT
<213> Artificial

<220>
<223> amino acid sequences of variable region of the heavy chain (VH) of humanized B-Ly1 antibody (B-HL13)

<400> 15

Glu Val Gln Leu Val Glu Ser Gly Gly Gly Val Val Lys Pro Gly Gly
1               5               10              15

Ser Leu Arg Leu Ser Cys Ala Ala Ser Gly Phe Thr Phe Ser Tyr Ser
            20              25              30

Trp Met Asn Trp Val Arg Gln Ala Pro Gly Lys Gly Leu Glu Trp Met
        35              40              45

Gly Arg Ile Phe Pro Gly Asp Gly Asp Thr Asp Tyr Asn Gly Lys Phe
    50              55              60

Lys Gly Arg Val Thr Ile Thr Ala Asp Lys Ser Thr Ser Thr Ala Tyr
65              70              75              80

Met Glu Leu Ser Ser Leu Arg Ser Glu Asp Thr Ala Val Tyr Tyr Cys
                85              90              95

Ala Arg Asn Val Phe Asp Gly Tyr Trp Leu Val Tyr Trp Gly Gln Gly

100                    105                    110

```
Thr Leu Val Thr Val Ser Ser
            115
```

<210> 16
<211> 119
<212> PRT
<213> Artificial

<220>
<223> amino acid sequences of variable region of the heavy chain (VH) of humanized B-Ly1 antibody (B-HL14)

<400> 16

```
Glu Val Gln Leu Val Glu Ser Gly Gly Gly Leu Lys Lys Pro Gly Gly
1               5               10                  15

Ser Leu Arg Leu Ser Cys Ala Ala Ser Gly Phe Thr Phe Ser Tyr Ser
            20              25                  30

Trp Met Asn Trp Val Arg Gln Ala Pro Gly Lys Gly Leu Glu Trp Met
            35              40                  45

Gly Arg Ile Phe Pro Gly Asp Gly Asp Thr Asp Tyr Asn Gly Lys Phe
        50              55                  60

Lys Gly Arg Val Thr Ile Thr Ala Asp Lys Ser Thr Ser Thr Ala Tyr
65                  70                  75                      80

Met Glu Leu Ser Ser Leu Arg Ser Glu Asp Thr Ala Val Tyr Tyr Cys
                85                  90                  95

Ala Arg Asn Val Phe Asp Gly Tyr Trp Leu Val Tyr Trp Gly Gln Gly
                100             105                 110

Thr Leu Val Thr Val Ser Ser
            115
```

<210> 17
<211> 119
<212> PRT
<213> Artificial

<220>
<223> amino acid sequences of variable region of the heavy chain (VH) of humanized B-Ly1 antibody (B-HL15)

<400> 17

```
Glu Val Gln Leu Val Glu Ser Gly Gly Gly Leu Val Lys Pro Gly Ser
1               5               10              15

Ser Leu Arg Leu Ser Cys Ala Ala Ser Gly Phe Thr Phe Ser Tyr Ser
            20              25              30

Trp Met Asn Trp Val Arg Gln Ala Pro Gly Lys Gly Leu Glu Trp Met
        35              40              45

Gly Arg Ile Phe Pro Gly Asp Gly Asp Thr Asp Tyr Asn Gly Lys Phe
    50              55              60

Lys Gly Arg Val Thr Ile Thr Ala Asp Lys Ser Thr Ser Thr Ala Tyr
65              70              75              80

Met Glu Leu Ser Ser Leu Arg Ser Glu Asp Thr Ala Val Tyr Tyr Cys
            85              90              95

Ala Arg Asn Val Phe Asp Gly Tyr Trp Leu Val Tyr Trp Gly Gln Gly
            100             105             110

Thr Leu Val Thr Val Ser Ser
            115
```

<210> 18
<211> 119
<212> PRT
<213> Artificial

<220>
<223> amino acid sequences of variable region of the heavy chain (VH) of humanized B-Ly1 antibody (B-HL16)

<400> 18

```
Glu Val Gln Leu Val Glu Ser Gly Gly Gly Leu Val Lys Pro Gly Gly
1               5               10              15

Ser Leu Arg Val Ser Cys Ala Ala Ser Gly Phe Thr Phe Ser Tyr Ser
            20              25              30

Trp Met Asn Trp Val Arg Gln Ala Pro Gly Lys Gly Leu Glu Trp Met
        35              40              45

Gly Arg Ile Phe Pro Gly Asp Gly Asp Thr Asp Tyr Asn Gly Lys Phe
    50              55              60
```

```
Lys Gly Arg Val Thr Ile Thr Ala Asp Lys Ser Thr Ser Thr Ala Tyr
65              70              75              80

Met Glu Leu Ser Ser Leu Arg Ser Glu Asp Thr Ala Val Tyr Tyr Cys
            85              90              95

Ala Arg Asn Val Phe Asp Gly Tyr Trp Leu Val Tyr Trp Gly Gln Gly
        100             105             110

Thr Leu Val Thr Val Ser Ser
        115
```

<210> 19
<211> 119
<212> PRT
<213> Artificial

<220>
<223> amino acid sequences of variable region of the heavy chain (VH) of humanized B-Ly1 antibody (B-HL17)

<400> 19

```
Glu Val Gln Leu Val Glu Ser Gly Gly Gly Leu Val Lys Pro Gly Gly
1               5               10              15

Ser Leu Arg Leu Ser Cys Ala Ala Ser Gly Phe Thr Phe Ser Tyr Ser
        20              25              30

Trp Met Asn Trp Val Arg Gln Ala Pro Gly Lys Gly Leu Glu Trp Met
        35              40              45

Gly Arg Ile Phe Pro Gly Asp Gly Asp Thr Asp Tyr Asn Gly Lys Phe
    50              55              60

Lys Gly Arg Val Thr Ile Thr Ala Asp Lys Ser Thr Ser Thr Ala Tyr
65              70              75              80

Met Glu Leu Ser Ser Leu Arg Ser Glu Asp Thr Ala Val Tyr Tyr Cys
            85              90              95

Ala Arg Asn Val Phe Asp Gly Tyr Trp Leu Val Tyr Trp Gly Gln Gly
        100             105             110

Thr Leu Val Thr Val Ser Ser
        115
```

<210> 20
<211> 115
<212> PRT

<213> Artificial

<220>
<223> amino acid sequences of variable region of the light chain (VL) of humanized B-Ly1 antibody B-KV1

<400> 20

```
Asp Ile Val Met Thr Gln Thr Pro Leu Ser Leu Pro Val Thr Pro Gly
1               5                   10                  15

Glu Pro Ala Ser Ile Ser Cys Arg Ser Ser Lys Ser Leu Leu His Ser
            20                  25                  30

Asn Gly Ile Thr Tyr Leu Tyr Trp Tyr Leu Gln Lys Pro Gly Gln Ser
            35                  40                  45

Pro Gln Leu Leu Ile Tyr Gln Met Ser Asn Leu Val Ser Gly Val Pro
        50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ala Gln Asn
                85                  90                  95

Leu Glu Leu Pro Tyr Thr Phe Gly Gly Gly Thr Lys Val Glu Ile Lys
            100                 105                 110

Arg Thr Val
        115
```

SEQUENCE LISTING

<110> F. Hoffmann-La Roche AG

<120> Combination therapy with type I and type II anti-CD20 antibodies

<130> 24509

<150> EP 07017337
<151> 2007-09-05

<160> 20

<170> PatentIn version 3.2

<210> 1
<211> 112
<212> PRT
<213> Mus sp.

<220>

<221> MISC_FEATURE
<223> amino acid sequence of variable region of the heavy chain (VH) of murine monoclonal anti-CD20 antibody B-Ly1

<400> 1

```
Gly Pro Glu Leu Val Lys Pro Gly Ala Ser Val Lys Ile Ser Cys Lys
1               5                   10                  15

Ala Ser Gly Tyr Ala Phe Ser Tyr Ser Trp Met Asn Trp Val Lys Leu
            20                  25                  30

Arg Pro Gly Gln Gly Leu Glu Trp Ile Gly Arg Ile Phe Pro Gly Asp
            35                  40                  45

Gly Asp Thr Asp Tyr Asn Gly Lys Phe Lys Gly Lys Ala Thr Leu Thr
        50                  55                  60

Ala Asp Lys Ser Ser Asn Thr Ala Tyr Met Gln Leu Thr Ser Leu Thr
65                  70                  75                  80

Ser Val Asp Ser Ala Val Tyr Leu Cys Ala Arg Asn Val Phe Asp Gly
                85                  90                  95

Tyr Trp Leu Val Tyr Trp Gly Gln Gly Thr Leu Val Thr Val Ser Ala
            100                 105                 110
```

<210> 2
<211> 103
<212> PRT
<213> Mus sp.

<220>
<221> MISC_FEATURE
<223> amino acid sequence of variable region of the light chain (VL) of murine monoclonal anti-CD20 antibody B-Ly1

<400> 2

```
Asn Pro Val Thr Leu Gly Thr Ser Ala Ser Ile Ser Cys Arg Ser Ser
1               5                   10                  15
```

```
Lys Ser Leu Leu His Ser Asn Gly Ile Thr Tyr Leu Tyr Trp Tyr Leu
            20                  25              30

Gln Lys Pro Gly Gln Ser Pro Gln Leu Leu Ile Tyr Gln Met Ser Asn
            35                  40              45

Leu Val Ser Gly Val Pro Asp Arg Phe Ser Ser Ser Gly Ser Gly Thr
            50                  55              60

Asp Phe Thr Leu Arg Ile Ser Arg Val Glu Ala Glu Asp Val Gly Val
65                  70                  75                  80

Tyr Tyr Cys Ala Gln Asn Leu Glu Leu Pro Tyr Thr Phe Gly Gly Gly
                85                  90                  95

Thr Lys Leu Glu Ile Lys Arg
                100
```

<210> 3
<211> 119
<212> PRT
<213> Artificial

<220>
<223> amino acid sequences of variable region of the heavy chain (VH) of humanized B-Ly1 antibody (B-HH2)

<400> 3

```
Gln Val Gln Leu Val Gln Ser Gly Ala Glu Val Lys Lys Pro Gly Ser
1               5               10              15

Ser Val Lys Val Ser Cys Lys Ala Ser Gly Tyr Ala Phe Ser Tyr Ser
            20                  25              30

Trp Met Asn Trp Val Arg Gln Ala Pro Gly Gln Gly Leu Glu Trp Met
            35                  40              45

Gly Arg Ile Phe Pro Gly Asp Gly Asp Thr Asp Tyr Asn Gly Lys Phe
            50                  55              60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95
```

```
        Ala Arg Asn Val Phe Asp Gly Tyr Trp Leu Val Tyr Trp Gly Gln Gly
                    100                 105                 110


        Thr Leu Val Thr Val Ser Ser
                    115
```

<210> 4
<211> 119
<212> PRT
<213> Artificial

<220>
<223> amino acid sequences of variable region of the heavy chain (VH) of humanized B-Ly1 antibody (B-HH3)

<400> 4

```
        Gln Val Gln Leu Val Gln Ser Gly Ala Glu Val Lys Lys Pro Gly Ser
        1               5                   10                  15


        Ser Val Lys Val Ser Cys Lys Ala Ser Gly Tyr Ala Phe Ser Tyr Ser
                    20                  25                  30


        Trp Met Asn Trp Val Arg Gln Ala Pro Gly Gln Gly Leu Glu Trp Met
                    35                  40                  45


        Gly Arg Ile Phe Pro Gly Asp Gly Asp Thr Asp Tyr Asn Gly Lys Phe
                    50                  55                  60


        Lys Gly Arg Val Thr Ile Thr Ala Asp Lys Ser Thr Ser Thr Ala Tyr
        65                  70                  75                  80


        Met Glu Leu Ser Ser Leu Arg Ser Glu Asp Thr Ala Val Tyr Leu Cys
                    85                  90                  95


        Ala Arg Asn Val Phe Asp Gly Tyr Trp Leu Val Tyr Trp Gly Gln Gly
                    100                 105                 110


        Thr Leu Val Thr Val Ser Ser
                    115
```

<210> 5
<211> 119
<212> PRT
<213> Artificial

<220>
<223> amino acid sequences of variable region of the heavy chain (VH) of humanized B-Ly1 antibody (B-HH4)

<400> 5

```
Gln Val Gln Leu Val Gln Ser Gly Ala Glu Val Lys Lys Pro Gly Ala
1               5               10              15

Ser Val Lys Val Ser Cys Lys Val Ser Gly Tyr Ala Phe Ser Tyr Ser
            20              25                  30

Trp Met Asn Trp Val Arg Gln Ala Pro Gly Gln Gly Leu Glu Trp Met
        35              40                  45

Gly Arg Ile Phe Pro Gly Asp Gly Asp Thr Asp Tyr Asn Gly Lys Phe
    50              55                  60

Lys Gly Arg Val Thr Ile Thr Ala Asp Lys Ser Thr Ser Thr Ala Tyr
65              70                  75                  80

Met Glu Leu Ser Ser Leu Arg Ser Glu Asp Thr Ala Val Tyr Tyr Cys
            85                  90                  95

Ala Arg Asn Val Phe Asp Gly Tyr Trp Leu Val Tyr Trp Gly Gln Gly
            100             105                 110

Thr Leu Val Thr Val Ser Ser
            115
```

<210> 6
<211> 119
<212> PRT
<213> Artificial

<220>
<223> amino acid sequences of variable region of the heavy chain (VH) of humanized B-Ly1 antibody (B-HH5)

<400> 6

```
Gln Val Gln Leu Val Gln Ser Gly Ala Glu Val Lys Lys Pro Gly Ser
1               5               10              15

Ser Val Lys Val Ser Cys Lys Ala Ser Gly Tyr Ala Phe Ser Tyr Ser
            20              25                  30

Trp Met Ser Trp Val Arg Gln Ala Pro Gly Gln Gly Leu Glu Trp Met
        35              40                  45

Gly Arg Ile Phe Pro Gly Asp Gly Asp Thr Asp Tyr Asn Gly Lys Phe
```

34

                50                          55                          60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asn Val Phe Asp Gly Tyr Trp Leu Val Tyr Trp Gly Gln Gly
                100                 105                 110

Thr Leu Val Thr Val Ser Ser
            115

<210> 7
<211> 119
<212> PRT
<213> Artificial

<220>
<223> amino acid sequences of variable region of the heavy chain (VH) of humanized B-Ly1 antibody (B-HH6)

<400> 7

Gln Val Gln Leu Val Gln Ser Gly Ala Glu Val Lys Lys Pro Gly Ser
1                   5                   10                  15

Ser Val Lys Val Ser Cys Lys Ala Ser Gly Tyr Ala Phe Ser Tyr Ser
                20                  25                  30

Trp Ile Asn Trp Val Arg Gln Ala Pro Gly Gln Gly Leu Glu Trp Met
            35                  40                  45

Gly Arg Ile Phe Pro Gly Asp Gly Asp Thr Asp Tyr Asn Gly Lys Phe
            50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asn Val Phe Asp Gly Tyr Trp Leu Val Tyr Trp Gly Gln Gly
                100                 105                 110

Thr Leu Val Thr Val Ser Ser
                115

<210> 8
<211> 119
<212> PRT
<213> Artificial

<220>
<223> amino acid sequences of variable region of the heavy chain (VH) of humanized B-Ly1 antibody (B-HH7)

<400> 8

```
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Ala Phe Ser Tyr Ser
            20                  25                  30

Trp Ile Ser Trp Val Arg Gln Ala Pro Gly Gln Gly Leu Glu Trp Met
        35                  40                  45

Gly Arg Ile Phe Pro Gly Asp Gly Asp Thr Asp Tyr Asn Gly Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asn Val Phe Asp Gly Tyr Trp Leu Val Tyr Trp Gly Gln Gly
            100                 105                 110

Thr Leu Val Thr Val Ser Ser
            115
```

<210> 9
<211> 119
<212> PRT
<213> Artificial

<220>
<223> amino acid sequences of variable region of the heavy chain (VH) of humanized B-Ly1 antibody (B-HH8)

<400> 9

```
Gln Val Gln Leu Val Gln Ser Gly Ala Glu Val Lys Lys Pro Gly Ala
1               5                   10                  15
```

```
Ser Val Lys Val Ser Cys Lys Ala Ser Gly Tyr Thr Phe Thr Tyr Ser
            20              25              30

Trp Met Asn Trp Val Arg Gln Ala Pro Gly Gln Gly Leu Glu Trp Met
            35              40              45

Gly Arg Ile Phe Pro Gly Asp Gly Asp Thr Asp Tyr Asn Gly Lys Phe
            50              55              60

Lys Gly Arg Val Thr Ile Thr Ala Asp Lys Ser Thr Ser Thr Ala Tyr
65              70              75              80

Met Glu Leu Ser Ser Leu Arg Ser Glu Asp Thr Ala Val Tyr Tyr Cys
                85              90              95

Ala Arg Asn Val Phe Asp Gly Tyr Trp Leu Val Tyr Trp Gly Gln Gly
            100             105             110

Thr Leu Val Thr Val Ser Ser
            115
```

<210> 10
<211> 119
<212> PRT
<213> Artificial

<220>
<223> amino acid sequences of variable region of the heavy chain (VH) of humanized B-Ly1 antibody (B-HH9)

<400> 10

```
Gln Val Gln Leu Val Gln Ser Gly Ala Glu Val Lys Lys Pro Gly Ala
1               5               10              15

Ser Val Lys Val Ser Cys Lys Ala Ser Gly Tyr Thr Phe Ser Tyr Ser
            20              25              30

Trp Met Asn Trp Val Arg Gln Ala Pro Gly Gln Gly Leu Glu Trp Met
            35              40              45

Gly Arg Ile Phe Pro Gly Asp Gly Asp Thr Asp Tyr Asn Gly Lys Phe
            50              55              60

Lys Gly Arg Val Thr Ile Thr Ala Asp Lys Ser Thr Ser Thr Ala Tyr
65              70              75              80
```

Met Glu Leu Ser Ser Leu Arg Ser Glu Asp Thr Ala Val Tyr Tyr Cys
                85                  90                  95

Ala Arg Asn Val Phe Asp Gly Tyr Trp Leu Val Tyr Trp Gly Gln Gly
            100              105              110

Thr Leu Val Thr Val Ser Ser
            115

<210> 11
<211> 119
<212> PRT
<213> Artificial

<220>
<223> amino acid sequences of variable region of the heavy chain (VH) of humanized B-Ly1 antibody (B-HL8)

<400> 11

Glu Val Gln Leu Val Glu Ser Gly Gly Gly Leu Val Lys Pro Gly Gly
1                   5                   10                  15

Ser Leu Arg Leu Ser Cys Ala Ala Ser Gly Phe Thr Phe Ser Tyr Ser
            20                  25                  30

Trp Met Asn Trp Val Arg Gln Ala Pro Gly Lys Gly Leu Glu Trp Val
            35                  40                  45

Gly Arg Ile Phe Pro Gly Asp Gly Asp Thr Asp Tyr Asn Gly Lys Phe
        50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asn Val Phe Asp Gly Tyr Trp Leu Val Tyr Trp Gly Gln Gly
            100              105              110

Thr Leu Val Thr Val Ser Ser
            115

<210> 12
<211> 119
<212> PRT
<213> Artificial

<220>
<223> amino acid sequences of variable region of the heavy chain (VH) of humanized B-Ly1 antibody (B-HL10)

<400> 12

```
Glu Val Gln Leu Val Glu Ser Gly Gly Gly Leu Val Lys Pro Gly Gly
1               5               10              15

Ser Leu Arg Leu Ser Cys Ala Ala Ser Gly Phe Ala Phe Ser Tyr Ser
            20              25              30

Trp Met Asn Trp Val Arg Gln Ala Pro Gly Lys Gly Leu Glu Trp Val
        35              40              45

Gly Arg Ile Phe Pro Gly Asp Gly Asp Thr Asp Tyr Asn Gly Lys Phe
        50              55              60

Lys Gly Arg Val Thr Ile Thr Ala Asp Lys Ser Thr Ser Thr Ala Tyr
65              70              75              80

Met Glu Leu Ser Ser Leu Arg Ser Glu Asp Thr Ala Val Tyr Tyr Cys
            85              90              95

Ala Arg Asn Val Phe Asp Gly Tyr Trp Leu Val Tyr Trp Gly Gln Gly
        100             105             110

Thr Leu Val Thr Val Ser Ser
        115
```

<210> 13
<211> 119
<212> PRT
<213> Artificial

<220>
<223> amino acid sequences of variable region of the heavy chain (VH) of humanized B-Ly1 antibody (B-HL11)

<400> 13

```
Gln Val Gln Leu Val Glu Ser Gly Gly Gly Leu Val Lys Pro Gly Gly
1               5               10              15

Ser Leu Arg Leu Ser Cys Ala Ala Ser Gly Phe Thr Phe Ser Tyr Ser
            20              25              30
```

```
Trp Met Asn Trp Val Arg Gln Ala Pro Gly Lys Gly Leu Glu Trp Val
        35              40                    45

Gly Arg Ile Phe Pro Gly Asp Gly Asp Thr Asp Tyr Asn Gly Lys Phe
    50              55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
            85                  90                      95

Ala Arg Asn Val Phe Asp Gly Tyr Trp Leu Val Tyr Trp Gly Gln Gly
        100                 105                 110

Thr Leu Val Thr Val Ser Ser
        115
```

<210> 14
<211> 119
<212> PRT
<213> Artificial

<220>
<223> amino acid sequences of variable region of the heavy chain (VH) of humanized B-Ly1 antibody (B-HL12)

<400> 14

```
Glu Val Gln Leu Val Glu Ser Gly Ala Gly Leu Val Lys Pro Gly Gly
1               5                   10                  15

Ser Leu Arg Leu Ser Cys Ala Ala Ser Gly Phe Thr Phe Ser Tyr Ser
        20                  25                  30

Trp Met Asn Trp Val Arg Gln Ala Pro Gly Lys Gly Leu Glu Trp Met
        35                  40                  45

Gly Arg Ile Phe Pro Gly Asp Gly Asp Thr Asp Tyr Asn Gly Lys Phe
    50              55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
            85                  90                      95
```

```
Ala Arg Asn Val Phe Asp Gly Tyr Trp Leu Val Tyr Trp Gly Gln Gly
            100                 105             110
```

```
Thr Leu Val Thr Val Ser Ser
            115
```

<210> 15
<211> 119
<212> PRT
<213> Artificial

<220>
<223> amino acid sequences of variable region of the heavy chain (VH) of humanized B-Ly1 antibody (B-HL13)

<400> 15

```
Glu Val Gln Leu Val Glu Ser Gly Gly Gly Val Val Lys Pro Gly Gly
1               5               10              15
```

```
Ser Leu Arg Leu Ser Cys Ala Ala Ser Gly Phe Thr Phe Ser Tyr Ser
            20              25              30
```

```
Trp Met Asn Trp Val Arg Gln Ala Pro Gly Lys Gly Leu Glu Trp Met
            35              40              45
```

```
Gly Arg Ile Phe Pro Gly Asp Gly Asp Thr Asp Tyr Asn Gly Lys Phe
        50              55              60
```

```
Lys Gly Arg Val Thr Ile Thr Ala Asp Lys Ser Thr Ser Thr Ala Tyr
65              70              75              80
```

```
Met Glu Leu Ser Ser Leu Arg Ser Glu Asp Thr Ala Val Tyr Tyr Cys
            85              90              95
```

```
Ala Arg Asn Val Phe Asp Gly Tyr Trp Leu Val Tyr Trp Gly Gln Gly
            100                 105             110
```

```
Thr Leu Val Thr Val Ser Ser
            115
```

<210> 16
<211> 119
<212> PRT
<213> Artificial

<220>
<223> amino acid sequences of variable region of the heavy chain (VH) of humanized B-Ly1 antibody (B-HL14)

<400> 16

```
Glu Val Gln Leu Val Glu Ser Gly Gly Gly Leu Lys Lys Pro Gly Gly
1               5               10              15

Ser Leu Arg Leu Ser Cys Ala Ala Ser Gly Phe Thr Phe Ser Tyr Ser
            20              25              30

Trp Met Asn Trp Val Arg Gln Ala Pro Gly Lys Gly Leu Glu Trp Met
        35              40              45

Gly Arg Ile Phe Pro Gly Asp Gly Asp Thr Asp Tyr Asn Gly Lys Phe
    50              55              60

Lys Gly Arg Val Thr Ile Thr Ala Asp Lys Ser Thr Ser Thr Ala Tyr
65              70              75              80

Met Glu Leu Ser Ser Leu Arg Ser Glu Asp Thr Ala Val Tyr Tyr Cys
            85              90              95

Ala Arg Asn Val Phe Asp Gly Tyr Trp Leu Val Tyr Trp Gly Gln Gly
            100             105             110

Thr Leu Val Thr Val Ser Ser
            115
```

<210> 17
<211> 119
<212> PRT
<213> Artificial

<220>
<223> amino acid sequences of variable region of the heavy chain (VH) of humanized B-Ly1 antibody (B-HL15)

<400> 17

```
Glu Val Gln Leu Val Glu Ser Gly Gly Gly Leu Val Lys Pro Gly Ser
1               5               10              15

Ser Leu Arg Leu Ser Cys Ala Ala Ser Gly Phe Thr Phe Ser Tyr Ser
            20              25              30

Trp Met Asn Trp Val Arg Gln Ala Pro Gly Lys Gly Leu Glu Trp Met
        35              40              45
```

```
Gly Arg Ile Phe Pro Gly Asp Gly Asp Thr Asp Tyr Asn Gly Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asn Val Phe Asp Gly Tyr Trp Leu Val Tyr Trp Gly Gln Gly
            100                 105                 110

Thr Leu Val Thr Val Ser Ser
            115
```

<210> 18
<211> 119
<212> PRT
<213> Artificial

<220>
<223> amino acid sequences of variable region of the heavy chain (VH) of humanized B-Ly1 antibody (B-HL16)

<400> 18

```
Glu Val Gln Leu Val Glu Ser Gly Gly Gly Leu Val Lys Pro Gly Gly
1               5               10                  15

Ser Leu Arg Val Ser Cys Ala Ala Ser Gly Phe Thr Phe Ser Tyr Ser
            20                  25                  30

Trp Met Asn Trp Val Arg Gln Ala Pro Gly Lys Gly Leu Glu Trp Met
        35                  40                  45

Gly Arg Ile Phe Pro Gly Asp Gly Asp Thr Asp Tyr Asn Gly Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asn Val Phe Asp Gly Tyr Trp Leu Val Tyr Trp Gly Gln Gly
            100                 105                 110

Thr Leu Val Thr Val Ser Ser
            115
```

<210> 19
<211> 119
<212> PRT
<213> Artificial

<220>
<223> amino acid sequences of variable region of the heavy chain (VH) of humanized B-Ly1 antibody (B-HL17)

<400> 19

```
Glu Val Gln Leu Val Glu Ser Gly Gly Gly Leu Val Lys Pro Gly Gly
1               5               10              15

Ser Leu Arg Leu Ser Cys Ala Ala Ser Gly Phe Thr Phe Ser Tyr Ser
            20              25              30

Trp Met Asn Trp Val Arg Gln Ala Pro Gly Lys Gly Leu Glu Trp Met
        35              40              45

Gly Arg Ile Phe Pro Gly Asp Gly Asp Thr Asp Tyr Asn Gly Lys Phe
    50              55              60

Lys Gly Arg Val Thr Ile Thr Ala Asp Lys Ser Thr Ser Thr Ala Tyr
65              70              75              80

Met Glu Leu Ser Ser Leu Arg Ser Glu Asp Thr Ala Val Tyr Tyr Cys
            85              90              95

Ala Arg Asn Val Phe Asp Gly Tyr Trp Leu Val Tyr Trp Gly Gln Gly
            100             105             110

Thr Leu Val Thr Val Ser Ser
            115
```

<210> 20
<211> 115
<212> PRT
<213> Artificial

<220>
<223> amino acid sequences of variable region of the light chain (VL) of humanized B-Ly1 antibody B-KV1

<400> 20

```
Asp Ile Val Met Thr Gln Thr Pro Leu Ser Leu Pro Val Thr Pro Gly
```

```
    1                 5                      10                        15

    Glu Pro Ala Ser Ile Ser Cys Arg Ser Ser Lys Ser Leu Leu His Ser
                20                      25              30

    Asn Gly Ile Thr Tyr Leu Tyr Trp Tyr Leu Gln Lys Pro Gly Gln Ser
                35                  40              45

    Pro Gln Leu Leu Ile Tyr Gln Met Ser Asn Leu Val Ser Gly Val Pro
                50                  55              60

    Asp Arg Phe Ser Gly Ser Gly Ser Gly Thr Asp Phe Thr Leu Lys Ile
    65                  70                  75                  80

    Ser Arg Val Glu Ala Glu Asp Val Gly Val Tyr Tyr Cys Ala Gln Asn
                    85                  90                  95

    Leu Glu Leu Pro Tyr Thr Phe Gly Gly Gly Thr Lys Val Glu Ile Lys
                100                 105                 110

    Arg Thr Val
                115
```

**Description**

The invention relates to the use of a type I anti-CD20 antibody for the manufacture of a medicament for the treatment of a CD20 expressing cancer, characterized in that said type I anti-CD20 antibody is co-administered with a type II anti-CD20 antibody, characterized in that said type I anti-CD20 antibody is rituximab, said type II anti-CD20 antibody is a humanized B-Ly1 antibody, at least 40% or more of the oligosaccharides of the Fc region of said type II anti-CD20 antibody are non-fucosylated and said CD20 expressing cancer is a B-Cell Non-Hodgkin's lymphoma (NHL), and wherein said humanized B-Ly1 antibody comprises a variable region of the heavy chain (VH) of SEQ ID No.7 and a variable region of the light chain (VL) of SEQ ID No. 20. The disclosure generally relates to the use of two different anti-CD20 antibodies for the manufacture of a medicament for the treatment of cancer, especially of CD20 expressing cancers.

**Background of the Invention**

The CD20 molecule (also called human B-lymphocyte-restricted differentiation antigen or Bp35) is a hydrophobic transmembrane protein with a molecular weight of approximately 35 kD located on pre-B and mature B lymphocytes (Valentine, M.A., et al. J. Biol. Chem. 264(19) (1989) 11282-11287; and Einfield, D.A., et al. EMBO J. 7(3) (1988) 711-717). CD20 is found on the surface of greater than 90% of B cells from peripheral blood or lymphoid organs and is expressed during early pre-B cell development and remains until plasma cell differentiation. CD20 is present on both normal B cells as well as malignant B cells. In particular, CD20 is expressed on greater than 90% of B cell non-Hodgltin's lymphomas (NHL) (Anderson, K.C., et al., Blood 63(6) (1984) 1424-1433) but is not found on hematopoietic stem cells, pro-B cells, normal plasma cells, or other normal tissues (Tedder, T.F., et al., J, Immunol. 135 (2) (1985) 973- 979).

The 85 amino acid carboxyl-terminal region of the CD20 protein is located within the cytoplasm. The length of this region contrasts with that of other B cell-specific surface structures such as IgM, IgD, and IgG heavy chains or histocompatibility antigens class I1 a or ß chains, which have relatively short intracytoplasmic regions of 3, 3, 28, 15, and 16 amino acids, respectively (Komaromy, M., et al., NAR 11 (1983) 6775-6785). Of the last 61 carboxyl-terminal amino acids, 21 are acidic residues, whereas only 2 are basic, indicating that this region has a strong net negative charge. The GenBank Accession No. is NP-690605. It is thought that CD20 might be involved in regulating an early step (s) in the activation and differentiation process of B cells (Tedder et al., Eur. J. Immunol. 25 Vol. 16 (1986) 881-887) and could function as a calcium ion channel (Tedder, T.F., et al., J. Cell. Biochem. 14D (1990)195).

There exist two different types of anti-CD20 antibodies differing significantly in their mode of CD20 binding and biological activities (Cragg, M.S., et al, Blood, 103 (2004) 2738-2743; and Cragg, M.S., et al, Blood, 101 (2003) 1045-1052). Type I antibodies, as Rituximab, are potent in complement mediated cytotoxicity, whereas type II antibodies, as Tositumomab (B1), 11B8 and AT80 or humanized B-Ly1 antibodies, effectively initiate target cell death via caspase-independent apoptosis with concomitant phosphatidylserine exposure.

The sharing common features of type I and type II anti-CD20 antibodies are summarized in **Table 1:**

Table 1: Properties of type I and type II anti-CD20 antibodies

| type I anti-CD20 antibodies | type II anti-CD20 antibodies |
|---|---|
| type I CD20 epitope | type II CD20 epitope |
| Localize CD20 to lipid rafts | Do not localize CD20 to lipid rafts |
| Increased CDC (if IgG1 isotype) | Decreased CDC (if IgG1 isotype) |
| ADCC activity (if IgG1 isotype) | ADCC activity(if IgG1 isotype) |
| Full binding capacity | Reduced binding capacity |
| Homotypic aggregation | Stronger homotypic aggregation |
| Apoptosis induction upon cross-linking | Strong cell death induction without cross-linking |

WO2004035607 relates to human monoclonal antibodies against CD20 and their use for treatment of diseases associated with CD20 expressing cells.

**Summary of the Invention**

The invention relates to the following combination therapy with type I and type II anti-CD20 antibodies:

2

[1]. Use of a type I anti-CD20 antibody for the manufacture of a medicament for the treatment of a CD20 expressing cancer, characterized in that said type I anti-CD20 antibody is co-administered with a type II anti-CD20 antibody, characterized in that said type I anti-CD20 antibody is rituximab, said type II anti-CD20 antibody is a humanized B-Ly1 antibody, at least 40% or more of the oligosaccharides of the Fc region of said type II anti-CD20 antibody are non-fucosylated and said CD20 expressing cancer is a B-Cell Non-Hodgkin's lymphoma (NHL), and wherein said humanized B-Ly1 antibody comprises a variable region of the heavy chain (VH) of SEQ ID No.7 and a variable region of the light chain (VL) of SEQ ID No. 20.

[2]. Use according to [I], characterized in that said type II anti-CD20 antibody has increased antibody dependent cellular cytotoxicity (ADCC).

[3]. A type I anti-CD20 antibody for use in the treatment of a CD20 expressing cancer characterized in that said type I anti-CD20 antibody is co-administered with a type II anti-CD20 antibody characterized in that said type I anti-CD20 antibody is rituximab, said type II anti-CD20 antibody is a humanized B-Ly1 antibody, at least 40% or more of the oligosaccharides of the Fc region of said type II anti-CD20 antibody are non-fucosylated and said CD20 expressing cancer is a B-Cell Non-Hodgkin's lymphoma (NHL), and wherein said humanized B-Ly1 antibody comprises a variable region of the heavy chain (VH) of SEQ ID No.7 and a variable region of the light chain (VL) of SEQ ID No. 20.

[4]. The type I anti-CD20 antibody according to [3] for use in the treatment of a CD20 expressing cancer according to [3], characterized in that said type II anti-CD20 antibody has increased antibody dependent cellular cytotoxicity (ADCC).

The disclosure generally comprises the use of a type I anti-CD20 antibody for the manufacture of a medicament for the treatment of a CD20 expressing cancer characterized in that said type I anti-CD20 antibody is co-administered with a type II anti-CD20 antibody.

The disclosure further comprises the use of a type I anti-CD20 antibody as first anti-CD20 antibody for the manufacture of a medicament for the treatment of a CD20 expressing cancer characterized in that

a) said first anti-CD20 antibody has a ratio of the binding capacities to CD20 on Raji cells (ATCC-No. CCL-86) of said first anti-CD20 antibody compared to rituximab of 0.8 to 1.2,

b) said first anti-CD20 antibody is co-administered with a type II anti-CD20 antibody as a second anti-CD20 antibody

c) said second anti-CD20 antibody has a ratio of the binding capacities to CD20 on Raji cells (ATCC-No. CCL-86) of said second anti-CD20 antibody compared to rituximab of 0.3 to 0.6.

The disclosure comprises the use of a type I anti-CD20 antibody for the manufacture of a medicament for the treatment of a patient suffering from a CD20 expressing cancer characterized in that said type I anti-CD20 antibody is co-administered with a type II anti-CD20 antibody.

The disclosure further comprises the use of a type I anti-CD20 antibody as first anti-CD20 antibody for the manufacture of a medicament for the treatment of a patient suffering from a CD20 expressing cancer characterized in that

a) said first anti-CD20 antibody has a ratio of the binding capacities to CD20 on Raji cells (ATCC-No. CCL-86) of said first anti-CD20 antibody compared to rituximab of 0.8 to 1.2,

b) said first anti-CD20 antibody is co-administered with a type II anti-CD20 antibody as a second anti-CD20 antibody

c) said second anti-CD20 antibody has a ratio of the binding capacities to CD20 on Raji cells (ATCC-No. CCL-86) of said second anti-CD20 antibody compared to rituximab of 0.3 to 0.6.

The disclosure further comprises a type I anti-CD20 antibody for the treatment of a CD20 expressing cancer characterized in that said type I anti-CD20 antibody is co-administered with a type II anti-CD20 antibody.

The disclosure further comprises a type I anti-CD20 antibody for the treatment of a patient suffering from a CD20 expressing cancer characterized in that said type I anti-CD20 antibody is co-administered with a type II anti-CD20 antibody.

Preferably the CD20 expressing cancer is a B-Cell Non-Hodgkin's lymphoma (NHL).

Preferably said first and second anti-CD20 antibodies (type I and type II) are monoclonal antibodies.

Preferably said type I anti-CD20 antibody is rituximab.

Preferably said type II anti-CD20 antibody is a humanized B-Ly1 antibody.

Preferably said type II anti-CD20 antibody has increased antibody dependent cellular cytotoxicity (ADCC).

Preferably said type I anti-CD20 antibody has a ratio of the binding capacities to CD20 on Raji cells (ATCC-No. CCL-86) of said first anti-CD20 antibody compared to rituximab of 0.9 to 1.1.

Preferably said type II anti-CD20 antibody has a ratio of the binding capacities to CD20 on Raji cells (ATCC-No. CCL-86) of said second anti-CD20 antibody compared to rituximab of 0.35 to 0.55, more preferably of 0.4 to 0.5.

In one preferred embodiment of the disclosure said type I anti-CD20 antibody is rituximab, said type II anti-CD20 antibody is a humanized B-Ly1 antibody and said CD20 expressing cancer is a B-Cell Non-Hodgkin's lymphoma (NHL).

The disclosure further comprises a kit comprising a type II anti-CD20 antibody and a type I anti-CD20 antibody for the combination treatment of a patient suffering from a CD20 expressing cancer.

Preferably the kit is characterized in that said type I anti-CD20 antibody is rituximab, said type II anti-CD20 antibody is a humanized B-Ly1 antibody and said CD20 expressing cancer is a B-Cell Non-Hodgkin's lymphoma (NHL).

## Detailed Description of the Invention

The term "antibody" encompasses the various forms of antibodies including but not being limited to whole antibodies, human antibodies, humanized antibodies and genetically engineered antibodies like monoclonal antibodies, chimeric antibodies or recombinant antibodies as well as fragments of such antibodies as long as the characteristic properties according to the disclosure are retained.

The terms "monoclonal antibody" or "monoclonal antibody composition" as used herein refer to a preparation of antibody molecules of a single amino acid composition. Accordingly, the term "human monoclonal antibody" refers to antibodies displaying a single binding specificity which have variable and constant regions derived from human germline immunoglobulin sequences. In one embodiment, the human monoclonal antibodies are produced by a hybridoma which includes a B cell obtained from a transgenic non-human animal, e.g. a transgenic mouse, having a genome comprising a human heavy chain transgene and a light human chain transgene fused to an immortalized cell.

Preferably said first and second anti-CD20 antibodies (type I and type II) are monoclonal antibodies.

The term "chimeric antibody" refers to a monoclonal antibody comprising a variable region, i.e., binding region, from one source or species and at least a portion of a constant region derived from a different source or species, usually prepared by recombinant DNA techniques. Chimeric antibodies comprising a murine variable region and a human constant region are especially preferred. Such murine/human chimeric antibodies are the product of expressed immunoglobulin genes comprising DNA segments encoding murine immunoglobulin variable regions and DNA segments encoding human immunoglobulin constant regions. Other forms of "chimeric antibodies" encompassed herein are those in which the class or subclass has been modified or changed from that of the original antibody. Such "chimeric" antibodies are also referred to as "class-switched antibodies." Methods for producing chimeric antibodies involve conventional recombinant DNA and gene transfection techniques now well known in the art. See, e.g., Morrison, S.L., et al., Proc. Natl. Acad Sci. USA 81 (1984) 6851-6855; US 5,202,238 and US 5,204,244.

The term "humanized antibody" refers to antibodies in which the framework or "complementarity determining regions" (CDR) have been modified to comprise the CDR of an immunoglobulin of different specificity as compared to that of the parent immunoglobulin. In a preferred embodiment, a murine CDR is grafted into the framework region of a human antibody to prepare the "humanized antibody." See, e.g., Riechmann, L., et al., Nature 332 (1988) 323-327; and Neuberger, M.S., et al., Nature 314 (1985) 268-270. Particularly preferred CDRs correspond to those representing sequences recognizing the antigens noted above for chimeric and bifunctional antibodies.

The term "human antibody", as used herein, is intended to include antibodies having variable and constant regions derived from human germline immunoglobulin sequences. Human antibodies are well-known in the state of the art (van Dijk, M.A., and van de Winkel, J.G., Curr. Opin. in Chemical Biology. 5 (2001) 368-374). Based on such technology, human antibodies against a great variety of targets can be produced. Examples of human antibodies are for example described in Kellermann, S. A., et al., Curr Opin Biotechnol. 13 (2002) 593-597.

The term "recombinant human antibody", as used herein, is intended to include all human antibodies that are prepared, expressed, created or isolated by recombinant means, such as antibodies isolated from a host cell such as a NS0 or CHO cell or from an animal (e.g. a mouse) that is transgenic for human immunoglobulin genes or antibodies expressed using a recombinant expression vector transfected into a host cell. Such recombinant human antibodies have variable and constant regions derived from human germline immunoglobulin sequences in a rearranged form. The recombinant human antibodies described herein have been subjected to in vivo somatic hypermutation. Thus, the amino acid sequences of the VH and VL regions of the recombinant antibodies are sequences that, while derived from and related to human germline VH and VL sequences, may not naturally exist within the human antibody germline repertoire in vivo.

As used herein, "specifically binding" or "binds specifically to" refers to an antibody specifically binding to the CD20 antigen. Preferably the binding affinity is of KD-value of $10^{-8}$ mol/l or lower, preferably $10^{-9}$ mol/l or lower (e.g. $10^{-10}$ mol/l), more preferably with a KD-value of $10^{-10}$ mol/l or lower (e.g. $10^{-12}$ mol/l). The binding affinity is determined with a standard binding assay, such as surface plasmon resonance technique (e.g. Biacore®) on CD20 expressing cells.

The term "nucleic acid molecule", as used herein, is intended to include DNA molecules and RNA molecules. A nucleic acid molecule may be single-stranded or double-stranded, but preferably is double-stranded DNA.

The "constant domains" are not involved directly in binding the antibody to an antigen but are involved in the effector functions (ADCC, complement binding, and CDC).

The "variable region" (variable region of a light chain (VL), variable region of a heavy chain (VH)) as used herein denotes each of the pair of light and heavy chains which is involved directly in binding the antibody to the antigen. The domains of variable human light and heavy chains have the same general structure and each domain comprises four framework (FR) regions whose sequences are widely conserved, connected by three "hypervariable regions" (or complementarity determining regions, CDRs). The framework regions adopt a b-sheet conformation and the CDRs may form loops connecting the b-sheet structure. The CDRs in each chain are held in their three-dimensional structure by the framework regions and form together with the CDRs from the other chain the antigen binding site. The antibody heavy and light chain CDR3 regions play a particularly important role in the binding specificity/affinity of the antibodies described herein and therefore provide a further object of the disclosure.

The terms "hypervariable region" or "antigen-binding portion of an antibody" when used herein refer to the amino acid residues of an antibody which are responsible for antigen-binding. The hypervariable region comprises amino acid residues from the "complementarity determining regions" or "CDRs". "Framework" or "FR" regions are those variable domain regions other than the hypervariable region residues as herein defined. Therefore, the light and heavy chains of an antibody comprise from N- to C-terminus the domains FR1, CDR1, FR2, CDR2, FR3, CDR3, and FR4. Especially, CDR3 of the heavy chain is the region which contributes most to antigen binding. CDR and FR regions are determined according to the standard definition of Kabat, E.A., et al., Sequences of Proteins of Immunological Interest, 5th Ed. Public Health Service, National Institutes of Health, Bethesda, MD. (1991) and/or those residues from a "hypervariable loop".

The terms "CD20" and "CD20 antigen" are used interchangeably herein, and include any variants, isoforms and species homologs of human CD20 which are naturally expressed by cells or are expressed on cells transfected with the CD20 gene. Binding of an antibody of the invention to the CD20 antigen mediate the killing of cells expressing CD20 (e.g., a tumor cell) by inactivating CD20. The killing of the cells expressing CD20 may occur by one or more of the following mechanisms: Cell death/apoptosis induction, ADCC and/or CDC.

Synonyms of CD20, as recognized in the art, include B-lymphocyte antigen CD20, B-lymphocyte surface antigen B1, Leu-16, Bp35, BM5, and LF5.

The term "anti-CD20 antibody" according to the invention is an antibody that binds specifically to CD20 antigen. Depending on binding properties and biological activities of anti-CD20 antibodies to the CD20 antigen, two types of anti-CD20 antibodies (type I and type II anti-CD20 antibodies) can be distinguished according to Cragg, M.S., et al, Blood 103 (2004) 2738-2743; and Cragg, M.S., et al Blood 101 (2003) 1045-1052, see Table 2.

**Table 2: Properties of type I and type II anti-CD20 antibodies**

| type I anti-CD20 antibodies | type II anti-CD20 antibodies |
|---|---|
| type I CD20 epitope | type II CD20 epitope |
| Localize CD20 to lipid rafts | Do not localize CD20 to lipid rafts |
| Increased CDC (if IgG1 isotype) | Decreased CDC (if IgG1 isotype) |
| ADCC activity (if IgG1 isotype) | ADCC activity(if IgG1 isotype) |
| Full binding capacity | Reduced binding capacity |
| Homotypic aggregation | Stronger homotypic aggregation |
| Apoptosis induction upon cross-linking | Strong cell death induction without cross-linking |

One essential property of type I and type II anti-CD20 antibody is their mode of binding. Thus type I and type II anti-CD20 antibody can be classified by the ratio of the binding capacities to CD20 on Raji cells (ATCC-No. CCL-86) of said anti-CD20 antibody compared to rituximab.

The type I anti-CD20 antibodies have a ratio of the binding capacities to CD20 on Raji cells (ATCC-No. CCL-86) of said anti-CD20 antibody compared to rituximab of 0.8 to 1.2, preferably of 0.9 to 1.1. Examples of such type I anti-CD20 antibodies include e.g. rituximab, IF5 IgG2a (ECACC, hybridoma; Press, O.W., et al., Blood 69/2 (1987) 584-591), HI47 IgG3 (ECACC, hybridoma), 2C6 IgG1 (as disclosed in WO 2005/103081), 2F2 IgG1 (as disclosed and WO 2004/035607 and WO 2005/103081) and 2H7 IgG1 (as disclosed in WO 2004/056312). Preferably said type I anti-CD20 antibody is a monoclonal antibody that binds to the same epitope as rituximab.

The type II anti-CD20 antibodies have a ratio of the binding capacities to CD20 on Raji cells (ATCC-No. CCL-86) of said anti-CD20 antibody compared to rituximab of 0.3 to 0.6, preferably of 0.35 to 0.55, more preferably 0.4 to 0.5. Examples of such type II anti-CD20 antibodies include e.g. tositumomab (B1 IgG2a), humanized B-Ly1 antibody IgG1 (a chimeric humanized IgG1 antibody as disclosed in WO 2005/044859), 11B8 IgG1 (as disclosed in WO 2004/035607), and AT80 IgG1. Preferably said type II anti-CD20 antibody is a monoclonal antibody that binds to the same epitope as humanized B-Ly1 antibody (as disclosed in WO 2005/044859).

The "ratio of the binding capacities to CD20 on Raji cells (ATCC-No. CCL-86) of an anti-CD20 antibodies compared to rituximab" is determined by direct immunofluorescence measurement (the mean fluorescent intensities (MFI) is measured) using said anti-CD20 antibody conjugated with Cy5 and rituximab conjugated with Cy5 in a FACSArray (Becton Dickinson) with Raji cells (ATCC-No. CCL-86), as described in Example No. 2, and calculated as follows:

$$\text{Ratio of the binding capacities to CD20 on Raji cells (ATCC-No. CCL-86)} =$$

$$\frac{\text{MFI(Cy5-anti-CD20 antibody)}}{\text{MFI(Cy5-rituximab)}} \times \frac{\text{Cy5-labeling ratio(Cy5-rituximab)}}{\text{Cy5-labeling ratio(Cy5-anti-CD20 antibody)}}$$

MFI is the mean fluorescent intensity. The "Cy5-labeling ratio" as used herein means number of Cy5-label molecules per molecule antibody.

Typically said type I anti-CD20 antibody has a ratio of the binding capacities to CD20 on Raji cells (ATCC-No. CCL-86) of said first anti-CD20 antibody compared to rituximab of 0.8 to 1.2, preferably 0.9 to 1.1.

Typically said type II anti-CD20 antibody has a ratio of the binding capacities to CD20 on Raji cells (ATCC-No. CCL-86) of said second anti-CD20 antibody compared to rituximab of 0.3 to 0.6, preferably 0.35 to 0.55, more preferably 0.4 to 0.5.

In a preferred embodiment said type II anti-CD20 antibody, preferably a humanized B-Ly1 antibody, has increased antibody dependent cellular cytotoxicity (ADCC).

By "antibody having increased antibody dependent cellular cytotoxicity (ADCC)" is meant an antibody, as that term is defined herein, having increased ADCC as determined by any suitable method known to those of ordinary skill in the art. One accepted in vitro ADCC assay is as follows:

   1) the assay uses target cells that are known to express the target antigen recognized by the antigen-binding region of the antibody;

   2) the assay uses human peripheral blood mononuclear cells (PBMCs), isolated from blood of a randomly chosen healthy donor, as effector cells;

   3) the assay is carried out according to following protocol:

      i) the PBMCs are isolated using standard density centrifugation procedures and are suspended at 5 X 106 cells/ml in RPMI cell culture medium;

      ii) the target cells are grown by standard tissue culture methods, harvested from the exponential growth phase with a viability higher than 90%, washed in RPMI cell culture medium, labeled with 100 micro-Curies of "CI-, washed twice with cell culture medium, and resuspended in cell culture medium at a density of 1 0' cells/ml;

      iii) 100 microliters of the final target cell suspension above are transferred to each well of a 96-well microtiter plate;

      iv) the antibody is serially-diluted from 4000 ng/ml to 0.04 ng/ml in cell culture medium and 50 microliters of the resulting antibody solutions are added to the target cells in the 96-well microtiter plate, testing in triplicate various antibody concentrations covering the whole concentration range above;

      v) for the maximum release (MR) controls, 3 additional wells in the plate containing the labeled target cells, receive 50 microliters of a 2% (VN) aqueous solution of non-ionic detergent (Nonidet, Sigma, St. Louis), instead of the antibody solution (point iv above);

      vi) for the spontaneous release (SR) controls, 3 additional wells in the plate containing the labeled target cells, receive 50 microliters of RPMI cell culture medium instead of the antibody solution (point iv above);

vii) the 96-well microtiter plate is then centrifuged at 50 x g for 1 minute and incubated for 1 hour at 4 C;

viii) 50 microliters of the PBMC suspension (point i above) are added to each well to yield an effector:target cell ratio of 25: 1 and the plates are placed in an incubator under 5% CO2 atmosphere at 37 C for 4 hours;

ix) the cell-free supernatant from each well is harvested and the experimentally released radioactivity (ER) is quantified using a gamma counter;

x) the percentage of specific lysis is calculated for each antibody concentration according to the formula (ER-MR)/(MR-SR) x 100, where ER is the average radioactivity quantified (see point ix above) for that antibody concentration, MR is the average radioactivity quantified (see point ix above) for the MR controls (see point V above), and SR is the average radioactivity quantified (see point ix above) for the SR controls (see point vi above);

4) "increased ADCC" is defined as either an increase in the maximum percentage of specific lysis observed within the antibody concentration range tested above, and/or a reduction in the concentration of antibody required to achieve one half of the maximum percentage of specific lysis observed within the antibody concentration range tested above. The increase in ADCC is relative to the ADCC, measured with the above assay, mediated by the same antibody, produced by the same type of host cells, using the same standard production, purification, formulation and storage methods, which are known to those skilled in the art, but that has not been produced by host cells engineered to overexpress GnTIII.

Said "increased ADCC" can be obtained by glycoengineering of said antibodies, that means enhance said natural, cell-mediated effector functions of monoclonal antibodies by engineering their oligosaccharide component as described in Umana, P., et al., Nature Biotechnol. 17 (1999) 176-180 and US 6,602,684.

The term "complement-dependent cytotoxicity (CDC)" refers to lysis of human tumor target cells by the antibody described herein in the presence of complement. CDC is measured preferably by the treatment of a preparation of CD20 expressing cells with an anti-CD20 antibody described herein in the presence of complement. CDC is found if the antibody induces at a concentration of 100 nM the lysis (cell death) of 20% or more of the tumor cells after 4 hours. The assay is performed preferably with $^{51}$Cr or Eu labeled tumor cells and measurement of released $^{51}$Cr or Eu. Controls include the incubation of the tumor target cells with complement but without the antibody.

Typically type I and type II anti-CD20 antibodies of the IgG1 isotype show characteristic CDC properties. Type I anti-CD20 antibodies have and increased CDC (if IgG1 isotype) and type II anti-CD20 antibodies have a decreased CDC (if IgG1 isotype) compared to each other. Preferably both type I and type II anti-CD20 antibodies are IgG1 isotype antibodies.

The "rituximab" antibody is a genetically engineered chimeric human gamma 1 murine constant domain containing monoclonal antibody directed against the human CD20 antigen. This chimeric antibody contains human gamma 1 constant domains and is identified by the name "C2B8" in US 5,736,137 (Andersen, et. al.), issued on April 17,1998, assigned to IDEC Pharmaceuticals Corporation. Rituximab is approved for the treatment of patients with relapsed or refracting lowgrade or follicular, CD20 positive, B cell non-Hodgkin's lymphoma. In vitro mechanism of action studies have shown that rituximab exhibits human complement--dependent cytotoxicity (CDC) (Reiff, M.E., et. al, Blood 83(2) 435-445 (1994)). Additionally, it exhibits significant activity in assays that measure antibody-dependent cellular cytotoxicity (ADCC).

The term "humanized B-Ly1 antibody" refers to humanized B-Ly1 antibody as disclosed in WO 2005/044859 and WO 2007/031875, which were obtained from the murine monoclonal anti-CD20 antibody B-Ly1 (variable region of the murine heavy chain (VH): SEQ ID NO: 1; variable region of the murine light chain (VL): SEQ ID NO: 2- see Poppema, S. and Visser, L., Biotest Bulletin 3 (1987) 131-139;) by chimerization with a human constant domain from IgG1 and following humanization (see WO 2005/044859 and WO 2007/031875). These "humanized B-Ly1 antibodies" are disclosed in detail in WO 2005/044859 and WO 2007/031875.

Preferably the "humanized B-Ly1 antibody" has variable region of the heavy chain (VH) selected from the group of SEQ ID NO.3 to SEQ ID No. 19 (B-HH2 to B-HH9, B-HL8, and B-HL10 to B-HL17 of WO 2005/044859 and WO 2007/031875). Especially preferred are Seq. ID No. 3, 4, 7, 9, 11, 13 and 15 (B-HH2, BHH-3, B-HH6, B-HHB, B-HL8, B-HL11 and B-HL13 of WO 2005/044859). Preferably the "humanized B-Ly1 antibody" has variable region of the light chain (VL) of SEQ ID No. 20 (B-KV1 of WO 2005/044859). Furthermore the humanized B-Ly1 antibody is preferably an IgG1 antibody. Preferably such humanized B-Ly1 antibodies are glycoengineered (GE) in the Fc region according to the procedures described in WO 2005/044859, WO 2004/065540, WO 2007/031875, Umana, P., et al., Nature Biotechnol. 17 (1999) 176-180 and WO 99/154342. Such glycoengineered humanized B-Ly1 antibodies have an altered pattern of glycosylation in the Fc region, preferably having a reduced level of fucose residues. Preferably at least 40% or more (in one embodiment between 40% and 60%, in another embodiment at least 50%, and in still another embodiment at least 70% or more) of the oligosaccharides of the Fc region are non-fucosylated. Furthermore the oligosaccharides of the Fc region are preferably bisected.

The disclosure comprises the use of a type I anti-CD20 antibody for the manufacture of a medicament for the treatment of a CD20 expressing cancer characterized in that said type I anti-CD20 antibody is co-administered with a type II anti-CD20 antibody.

The disclosure comprises the use of a type I anti-CD20 antibody for the manufacture of a medicament for the treatment of a patient suffering from a CD20 expressing cancer characterized in that said type I anti-CD20 antibody is co-administered with a type II anti-CD20 antibody.

Preferably the use is characterized in that said type I anti-CD20 antibody is rituximab, said type II anti-CD20 antibody is a humanized B-Ly1 antibody and said CD20 expressing cancer is a B-Cell Non-Hodgkin's lymphoma (NHL).

The oligosaccharide component can significantly affect properties relevant to the efficacy of a therapeutic glycoprotein, including physical stability, resistance to protease attack, interactions with the immune system, pharmacokinetics, and specific biological activity. Such properties may depend not only on the presence or absence, but also on the specific structures, of oligosaccharides. Some generalizations between oligosaccharide structure and glycoprotein function can be made. For example, certain oligosaccharide structures mediate rapid clearance of the glycoprotein from the bloodstream through interactions with specific carbohydrate binding proteins, while others can be bound by antibodies and trigger undesired immune reactions. (Jenkins, N., et al., Nature Biotechnol. 14 (1996) 975-81).

Mammalian cells are the preferred hosts for production of therapeutic glycoproteins, due to their capability to glycosylate proteins in the most compatible form for human application. (Cumming, D.A., et al., Glycobiology 1 (1991) 115-30; Jenkins, N., et al., Nature Biotechnol. 14 (1996) 975-81). Bacteria very rarely glycosylate proteins, and like other types of common hosts, such as yeasts, filamentous fungi, insect and plant cells, yield glycosylation patterns associated with rapid clearance from the blood stream, undesirable immune interactions, and in some specific cases, reduced biological activity. Among mammalian cells, Chinese hamster ovary (CHO) cells have been most commonly used during the last two decades. In addition to giving suitable glycosylation patterns, these cells allow consistent generation of genetically stable, highly productive clonal cell lines. They can be cultured to high densities in simple bioreactors using serumfree media, and permit the development of safe and reproducible bioprocesses. Other commonly used animal cells include baby hamster kidney (BHK) cells, NSO- and SP2/0-mouse myeloma cells. More recently, production from transgenic animals has also been tested. (Jenkins, N., et al., Nature Biotechnol. 14 (1996) 975-81.

All antibodies contain carbohydrate structures at conserved positions in the heavy chain constant regions, with each isotype possessing a distinct array of N-linked carbohydrate structures, which variably affect protein assembly, secretion or functional activity. (Wright, A., and Morrison, S. L., Trends Biotech. 15 (1997) 26-32). The structure of the attached N-linked carbohydrate varies considerably, depending on the degree of processing, and can include highmannose, multiply-branched as well as biantennary complex oligosaccharides. (Wright, A., and Morrison, S. L., Trends Biotech. 15 (1997) 26-32). Typically, there is heterogeneous processing of the core oligosaccharide structures attached at a particular glycosylation site such that even monoclonal antibodies exist as multiple glycoforms. Likewise, it has been shown that major differences in antibody glycosylation occur between cell lines, and even minor differences are seen for a given cell line grown under different culture conditions. (Lifely, M. R. et al., Glycobiology 5(8) (1995) 813-22).

One way to obtain large increases in potency, while maintaining a simple production process and potentially avoiding significant, undesirable side effects, is to enhance the natural, cell-mediated effector functions of monoclonal antibodies by engineering their oligosaccharide component as described in Umana, P., et al., Nature Biotechnol. 17 (1999) 176-180 and US 6,602,684. IgG1 type antibodies, the most commonly used antibodies in cancer immunotherapy, are glycoproteins that have a conserved N-linked glycosylation site at Asn297 in each CH2 domain. The two complex biantennary oligosaccharides attached to Asn297 are buried between the CH2 domains, forming extensive contacts with the polypeptide backbone, and their presence is essential for the antibody to mediate effector functions such as antibody dependent cellular cytotoxicity (ADCC) (Lifely, M. R., et al., Glycobiology 5: 813-822 (1995); Jefferis, R., et al., Immunol. Rev. 163: 59-76 (1998); Wright, A. and Morrison, S. L., Trends Biotechnol. 15: 26-32 (1997)).

It was previously shown that overexpression in Chinese hamster ovary (CHO) cells of ß(1,4)-N-acetylglucosaminyltransferase I11 ("GnTII 17y), a glycosyltransferase catalyzing the formation of bisected oligosaccharides, significantly increases the in vitro ADCC activity of an antineuroblastoma chimeric monoclonal antibody (chCE7) produced by the engineered CHO cells. (see Umana, P., et al., Nature Biotechnol. 17 (1999) 176-180; and WO 99/154342).

The antibody chCE7 belongs to a large class of unconjugated monoclonal antibodies which have high tumor affinity and specificity, but have too little potency to be clinically useful when produced in standard industrial cell lines lacking the GnTIII enzyme (Umana, P., et al., Nature Biotechnol. 17 (1999) 176-180).That study was the first to show that large increases of ADCC activity could be obtained by engineering the antibody producing cells to express GnTIII, which also led to an increase in the proportion of constant region (Fc)-associated, bisected oligosaccharides, including bisected, non-fucosylated oligosaccharides, above the levels found in naturally-occurring antibodies.

The term "expression of the CD20" antigen is intended to indicate an significant level of expression of the CD20 antigen in a cell, preferably on the cell surface of a T- or B- Cell, more preferably a B-cell, from a tumor or cancer, respectively, preferably a non-solid tumor. Patients having a "CD20 expressing cancer" can be determined by standard assays known in the art. E.g. CD20 antigen expression is measured using immunohistochemical (IHC) detection, FACS or via PCR-based detection of the corresponding mRNA.

The term "CD20 expressing cancer" as used herein refers preferably to lymphomas (preferably B-Cell Non-Hodgkin's lymphomas (NHL)) and lymphocytic leukemias. Such lymphomas and lymphocytic leukemias include e.g. a) follicular lymphomas, b) Small Non-Cleaved Cell Lymphomas/ Burkitt's lymphoma (including endemic Burkitt's lymphoma, sporadic Burkitt's lymphoma and Non-Burkitt's lymphoma) c) marginal zone lymphomas (including extranodal marginal zone B cell lymphoma (Mucosa-associated lymphatic tissue lymphomas, MALT), nodal marginal zone B cell lymphoma and splenic marginal zone lymphoma), d) Mantle cell lymphoma (MCL), e) Large Cell Lymphoma (including B-cell diffuse large cell lymphoma (DLCL), Diffuse Mixed Cell Lymphoma, Immunoblastic Lymphoma, Primary Mediastinal B-Cell Lymphoma, Angiocentric Lymphoma-Pulmonary B-Cell Lymphoma) f) hairy cell leukemia, g ) lymphocytic lymphoma, waldenstrom's macroglobulinemia, h) acute lymphocytic leukemia (ALL), chronic lymphocytic leukemia (CLL)/ small lymphocytic lymphoma (SLL), B-cell prolymphocytic leukemia, i) plasma cell neoplasms, plasma cell myeloma, multiple myeloma, plasmacytoma j) Hodgkin's disease.

Preferably the CD20 expressing cancer is a B-Cell Non-Hodgkin's lymphomas (NHL). Especially the CD20 expressing cancer a Mantle cell lymphoma (MCL), acute lymphocytic leukemia (ALL), chronic lymphocytic leukemia (CLL), B-cell diffuse large cell lymphoma (DLCL), Burkitt's lymphoma, hairy cell leukemia, follicular lymphoma, multiple myeloma, marginal zone lymphoma, post transplant lymphoproliferative disorder (PTLD), HIV associated lymphoma, waldenstrom's macroglobulinemia, or primary CNS lymphoma.

The term "a method of treating" or its equivalent, when applied to, for example, cancer refers to a procedure or course of action that is designed to reduce or eliminate the number of cancer cells in a patient, or to alleviate the symptoms of a cancer. "A method of treating" cancer or another proliferative disorder does not necessarily mean that the cancer cells or other disorder will, in fact, be eliminated, that the number of cells or disorder will, in fact, be reduced, or that the symptoms of a cancer or other disorder will, in fact, be alleviated. Often, a method of treating cancer will be performed even with a low likelihood of success, but which, given the medical history and estimated survival expectancy of a patient, is nevertheless deemed to induce an overall beneficial course of action.

The terms "co-administration" or "co-administering " refer to the administration of said first and second anti-CD20 antibody as one single formulation or as two separate formulations. The co-administration can be simultaneous or sequential in either order, wherein preferably there is a time period while both (or all) active agents simultaneously exert their biological activities. If one single formulation is used, both anti-CD20 antibodies are co-administered simultaneously. If two separate formulations (one for the first anti-CD20 antibody and one for the second anti-CD20 antibody) are used, said first and second anti-CD20 antibody are co-administered either simultaneously (e.g. through one single continuous infusion or through two separate continuous infusions at the same time) or sequentially. When both antibodies are co-administered sequentially the dose is administered either on the same day in two separate administrations, e.g. two separate continuous infusions at different times, or one of the antibodies is administered on day 1 and the second antibody is co-administered on day 2 to day 7, preferably on day 2 to 4. Thus the term "sequentially" means within 7 days after the dose of the first antibody, preferably within 4 days after the dose of the first antibody; and the term "simultaneously" means at the same time. The terms "co-administration" with respect to the maintenance doses of the anti-CD20 antibodies mean that the maintenance doses can be either co-administered simultaneously, e.g. during one continuous infusion, if the treatment cycle is appropriate for both antibodies, e.g. every week. Or the maintenance doses are co-administered sequentially, either within one or within several days, e.g. the maintenance dose of one of the antibodies is administered approximately every week, and the maintenance dose of the second antibodies is co-administered also every 2 weeks. Also other treatment cycles /usually e.g. from 3 days up to several weeks, may be used for both antibodies.

It is self-evident that the antibodies are administered to the patient in therapeutically effective amount which is the amount of the subject compound or combination that will elicit the biological or medical response of a tissue, system, animal or human that is being sought by the researcher, veterinarian, medical doctor or other clinician.

The amount of co-administration of said first and second anti-CD20 antibody and the timing of co-administration will depend on the type (species, gender, age, weight, etc.) and condition of the patient being treated and the severity of the disease or condition being treated. Said first and second anti-CD20 antibody are suitably co-administered to the patient at one time or over a series of treatments. Depending on the type and severity of the disease, about 1 $\mu$g /kg to 50 mg/kg (e.g. 0.1-20 mg/kg) of said first or second anti-CD20 antibody is an initial candidate dosage for co-administration to the patient, whether, for example, by one or more separate administrations, or by continuous infusion. In one embodiment, the initial infusion time for said first or second anti-CD20 antibody may be longer than subsequent infusion times, for instance approximately 90 minutes for the initial infusion, and approximately 30 minutes for subsequent infusions (if the initial infusion is well tolerated).

The preferred dosage of said first or second anti-CD20 antibody will be in the range from about 0.05mg/kg to about 30mg/kg. Thus, one or more doses of about 0.5mg/kg, 2.0mg/kg, 4.0mg/kg, 10 mg/kg or 30mg/kg (or any combination thereof) may be co-administered to the patient. Depending on the on the type (species, gender, age, weight, etc.) and condition of the patient and on the type of anti-CD20 antibody , the dosage of said first can differ from the dosage of the second anti-CD20 antibody. Such doses may be co-administered daily or intermittently, e.g. every third to six day or even every one to three weeks. An initial higher loading dose, followed by one or more lower doses may be administered.

In a preferred embodiment, the medicament is useful for preventing or reducing metastasis or further dissemination in such a patient suffering from CD20 expressing cancer. The medicament is useful for increasing the duration of survival of such a patient, increasing the progression free survival of such a patient, increasing the duration of response, resulting in a statistically significant and clinically meaningful improvement of the treated patient as measured by the duration of survival, progression free survival, response rate or duration of response. In a preferred embodiment, the medicament is useful for increasing the response rate in a group of patients.

In the context of this invention, additional other cytotoxic, chemotherapeutic or anti-cancer agents, or compounds that enhance the effects of such agents may be used in the anti-CD20 antibody combination treatment of CD20 expressing cancer. Preferably the anti-CD20 antibody combination treatment is used without such additional cytotoxic, chemotherapeutic or anti-cancer agents, or compounds that enhance the effects of such agents.

Such agents include, for example: alkylating agents or agents with an alkylating action, such as cyclophosphamide (CTX; e.g. cytoxan®), chlorambucil (CHL; e.g. leukeran®), cisplatin (CisP; e.g. platinol®) busulfan (e.g. myleran®), melphalan, carmustine (BCNU), streptozotocin, triethylenemelamine (TEM), mitomycin C, and the like; anti-metabolites, such as methotrexate (MTX), etoposide (VP16; e.g. vepesid®), 6-mercaptopurine (6MP), 6-thiocguanine (6TG), cytarabine (Ara-C), 5-fluorouracil (5-FU), capecitabine (e.g. Xeloda®), dacarbazine (DTIC), and the like; antibiotics, such as actinomycin D, doxorubicin (DXR; e.g. adriamycin®), daunorubicin (daunomycin), bleomycin, mithramycin and the like; alkaloids, such as vinca alkaloids such as vincristine (VCR), vinblastine, and the like; and other antitumor agents, such as paclitaxel (e.g. taxol®) and paclitaxel derivatives, the cytostatic agents, glucocorticoids such as dexamethasone (DEX; e.g. decadron®) and corticosteroids such as prednisone, nucleoside enzyme inhibitors such as hydroxyurea, amino acid depleting enzymes such as asparaginase, leucovorin and other folic acid derivatives, and similar, diverse antitumor agents. The following  agents may also be used as additional agents: arnifostine (e.g. ethyol®), dactinomycin, mechlorethamine (nitrogen mustard), streptozocin, cyclophosphamide, lomustine (CCNU), doxorubicin lipo (e.g. doxil®), gemcitabine (e.g. gemzar®), daunorubicin lipo (e.g. daunoxome®), procarbazine, mitomycin, docetaxel (e.g. taxotere®), aldesleukin, carboplatin, oxaliplatin, cladribine, camptothecin, CPT 11 (irinotecan), 10-hydroxy 7-ethyl-camptothecin (SN38), floxuridine, fludarabine, ifosfamide, idarubicin, mesna, interferon beta, interferon alpha, mitoxantrone, topotecan, leuprolide, megestrol, melphalan, mercaptopurine, plicamycin, mitotane, pegaspargase, pentostatin, pipobroman, plicamycin, tamoxifen, teniposide, testolactone, thioguanine, thiotepa, uracil mustard, vinorelbine, chlorambucil. Preferably the anti-CD20 antibody combination treatment is used without such additional agents.

The use of the cytotoxic and anticancer agents described above as well as antiproliferative target-specific anticancer drug like protein kinase inhibitors in chemotherapeutic regimens is generally well characterized in the cancer therapy arts, and their use herein falls under the same considerations for monitoring tolerance and effectiveness and for controlling administration routes and dosages, with some adjustments. For example, the actual dosages of the cytotoxic agents may vary depending upon the patient's cultured cell response determined by using histoculture methods. Generally, the dosage will be reduced compared to the amount used in the absence of additional other agents.

Typical dosages of an effective cytotoxic agent can be in the ranges recommended by the manufacturer, and where indicated by in vitro responses or responses in animal models, can be reduced by up to about one order of magnitude concentration or amount. Thus, the actual dosage will depend upon the judgment of the physician, the condition of the patient, and the effectiveness of the therapeutic method based on the in vitro responsiveness of the primary cultured malignant cells or histocultured tissue sample, or the responses observed in the appropriate animal models.

In the context of this disclosure, an effective amount of ionizing radiation may be carried out and/or a radiopharmaceutical may be used in addition to the anti-CD20 antibody combination treatment of CD20 expressing cancer. The source of radiation can be either external or internal to the patient being treated. When the source is external to the patient, the therapy is known as external beam radiation therapy (EBRT). When the source of radiation is internal to the patient, the  treatment is called brachytherapy (BT). Radioactive atoms for use in the context of this invention can be selected from the group including, but not limited to, radium, cesium-137, iridium-192, americium-241, gold-198, cobalt-57, copper-67, technetium-99, iodine-123, iodine-131, and indium-111. Is also possible to label the antibody with such radioactive isotopes. Preferably the anti-CD20 antibody combination treatment is used without such ionizing radiation.

Radiation therapy is a standard treatment for controlling unresectable or inoperable tumors and/or tumor metastases. Improved results have been seen when radiation therapy has been combined with chemotherapy. Radiation therapy is based on the principle that high-dose radiation delivered to a target area will result in the death of reproductive cells in both tumor and normal tissues. The radiation dosage regimen is generally defined in terms of radiation absorbed dose (Gy), time and fractionation, and must be carefully defined by the oncologist. The amount of radiation a patient receives will depend on various considerations, but the two most important are the location of the tumor in relation to other critical structures or organs of the body, and the extent to which the tumor has spread. A typical course of treatment for a patient undergoing radiation therapy will be a treatment schedule over a 1 to 6 week period, with a total dose of between 10 and 80 Gy administered to the patient in a single daily fraction of about 1.8 to 2.0 Gy, 5 days a week. In a preferred embodiment described herein there is synergy when tumors in human patients are treated with the combination treatment described herein and radiation. In other words, the inhibition of tumor growth by means of the agents comprising the combination of the invention is enhanced when combined with radiation, optionally with additional chemotherapeutic or anticancer agents. Parameters of adjuvant radiation therapies are, for example, contained in WO 99/60023.

The antibodies are administered to a patient according to known methods, by intravenous administration as a bolus or by continuous infusion over a period of time, by intramuscular, intraperitoneal, intracerobrospinal, subcutaneous, intraarticular, intrasynovial, or intrathecal routes. Intravenous or subcutaneous administration of the antibodies is preferred.

The disclosure further comprises a kit characterized in comprising a container, a composition within the container comprising said type I and type II anti-CD20 antibody , either in the form of one single or two separate formulations, and a package insert instructing the user of the composition to administer said type I and type II anti-CD20 antibody to a patient suffering from CD20 expressing cancer.

Preferably the kit is characterized in that said type I anti-CD20 antibody is rituximab, said type II anti-CD20 antibody is a humanized B-Ly1 antibody and said CD20 expressing cancer is a B-Cell Non-Hodgkin's lymphoma (NHL).

The term "package insert" refers to instructions customarily included in commercial packages of therapeutic products, which may include information about the indications, usage, dosage, administration, contraindications and/or warnings concerning the use of such therapeutic products.

In a preferred embodiment, the article of manufacture containers may further include a pharmaceutically acceptable carrier. The article of manufacture may further include a sterile diluent, which is preferably stored in a separate additional container.

As used herein, a "pharmaceutically acceptable carrier" is intended to include any and all material compatible with pharmaceutical administration including solvents, dispersion media, coatings, antibacterial and antifungal agents, isotonic and absorption delaying agents, and other materials and compounds compatible with pharmaceutical administration. Except insofar as any conventional media or agent is incompatible with the active compound, use thereof in the compositions of the invention is contemplated. Supplementary active compounds can also be incorporated into the compositions.

Pharmaceutical Formulations

Therapeutic formulations of the antibodies used herein are prepared for storage by mixing an antibody having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG).

The formulations described herein may be two separate formulations for each of the anti-CD20 antibodies. Alternatively the formulation herein may also contain both antibodies in one formulation.

Additionally, the composition may further comprise a chemotherapeutic agent, cytotoxic agent, cytokine, growth inhibitory agent or anti-angiogenic agent. Such molecules are suitably present in combination in amounts that are effective for the purpose intended.

The active ingredients may also be entrapped in microcapsules prepared, for example, by coacervation techniques or by interracial polymerization, for example, hydroxymethylcellulose or gelatin-microcapsules and poly- (methylmethacylate) microcapsules, respectively, in colloidal drug delivery systems (for example, liposomes, albumin microspheres, microemulsions, nano- particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides (US 3,773,919), copolymers of L-glutamic acid and gamma-ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid.

The formulations to be used for in vivo administration must be sterile. This is readily accomplished by filtration through sterile filtration membranes.

The disclosure further comprises a type I anti-CD20 antibody for the treatment of a CD20 expressing cancer characterized in that said type I anti-CD20 antibody is co-administered with a type II anti-CD20 antibody.

The disclosure further comprises a type I anti-CD20 antibody for the treatment of a patient suffering from a CD20 expressing cancer characterized in that said type I anti-CD20 antibody is co-administered with a type II anti-CD20 antibody.

In one preferred embodiment described herein said type I anti-CD20 antibody is rituximab, said type 11 anti-CD20 antibody is a humanized B-Ly1 antibody and said CD20 expressing cancer is a B-Cell Non-Hodgkin's lymphoma (NHL).

The disclosure further comprises a type I anti-CD20 antibody for the treatment of a CD20 expressing cancer or of a patient suffering from a CD20 expressing cancer characterized in that

a) said type I anti-CD20 antibody has a ratio of the binding capacities to CD20 on Raji cells (ATCC-No. CCL-86) of said type I anti-CD20 antibody compared to rituximab of 0.8 to 1.2,

b) said type I anti-CD20 antibody is co-administered with a type II anti-CD20 antibody, and

c) said type II anti-CD20 antibody has a ratio of the binding capacities to CD20 on Raji cells (ATCC-No. CCL-86) of said type II anti-CD20 antibody compared to rituximab of 0.3 to 0.6.

Preferably the CD20 expressing cancer is a B-cell Non-Hodgkin's lymphoma (NHL).

Preferably said type I anti-CD20 antibody is rituximab.

Preferably said type II anti-CD20 antibody is a humanized B-Ly1 antibody.

Preferably said type II anti-CD20 antibody has increased antibody dependent cellular cytotoxicity (ADCC).

The following examples and figures are provided to aid the understanding of the invention.

## Sequence Listing

| SEQ ID NO: 1 | amino acid sequence of variable region of the heavy chain (VH) of murine monoclonal anti-CD20 antibody B-Ly1. |
| SEQ ID NO: 2 | amino acid sequence of variable region of the light chain (VL) of murine monoclonal anti-CD20 antibody B-Ly1. |
| SEQ ID NO: 3-19 | amino acid sequences of variable region of the heavy chain (VH) of humanized B-Ly1 antibodies (B-HH2 to B-HH9, B-HL8, and B-HL10 to B-HL17) |
| SEQ ID NO: 20 | amino acid sequences of variable region of the light chain (VL) of humanized B-Ly1 antibody B-KV1 |

## Description of the -Figures

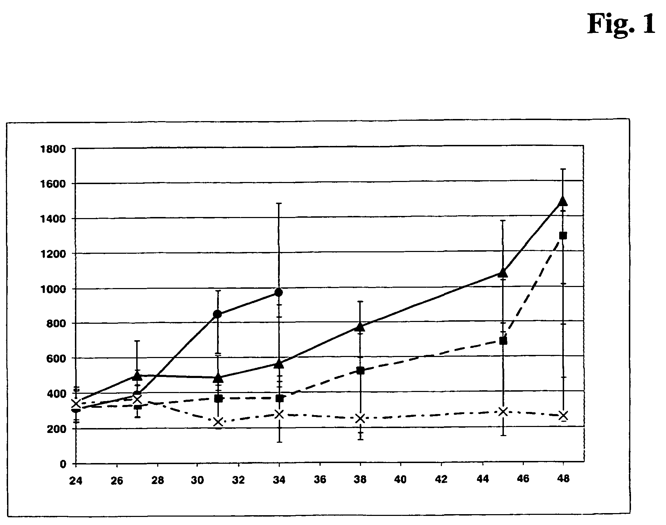

**Figure 1** Antitumor activity of combined treatment of a type I anti-CD20 antibody (rituximab) having a ratio of the binding capacities to CD20 on Raji cells (ATCC-No. CCL-86) of said type I anti-CD20 antibody compared to rituximab of 1.0, with a type II anti-CD20 antibody (B-HH6-B-KV1 GE) having a ratio of the binding capacities to CD20 on Raji cells (ATCC-No. CCL-86) of said type II anti-CD20 antibody compared to rituximab of 0.44, on OCI-Ly18 human Non-Hodgkin-Lymphoma (NHL). Mean values of tumor volume [mm$^3$] plotted on the $\gamma$-axis; number of days after injection of tumor cells plotted on the x-axis. Legend: A)Vehicle (*circles* ), B) rituximab 30 mg/kg i.v. once weekly (*triangles*). C) humanized B-ly1 (B-HH6-B-KV1 GE) 30 mg/kg once weekly (*squares*) and D) rituximab co-administered with B-HH6-B-KV1 GE (each 30 mg/kg once weekly) (crosses)

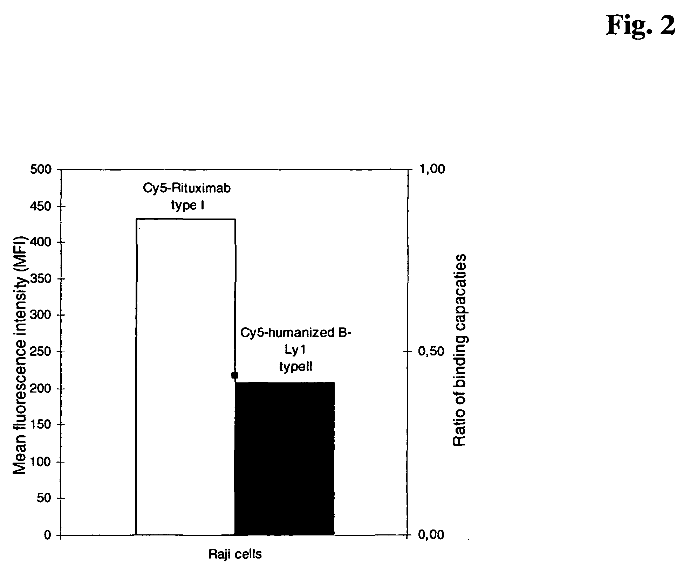

**Figure 2**    Mean Fluorescence Intensity (MFI, left y-axis) of type I anti-CD20 antibody (Cy5-rituximab = white bar) and type II anti-CD20 antibody (Cy5 humanized B-Ly1 B-HH6-B-KV1 GE = black bar) on Raji cells (ATCC-No. CCL-86) ; Ratio of the binding capacities to CD20 of type I anti-CD20 antibody (rituximab) and type II anti-CD20 antibody (B-HH6-B-KV1 GE) compared to rituximab (scaled on right y-axis)

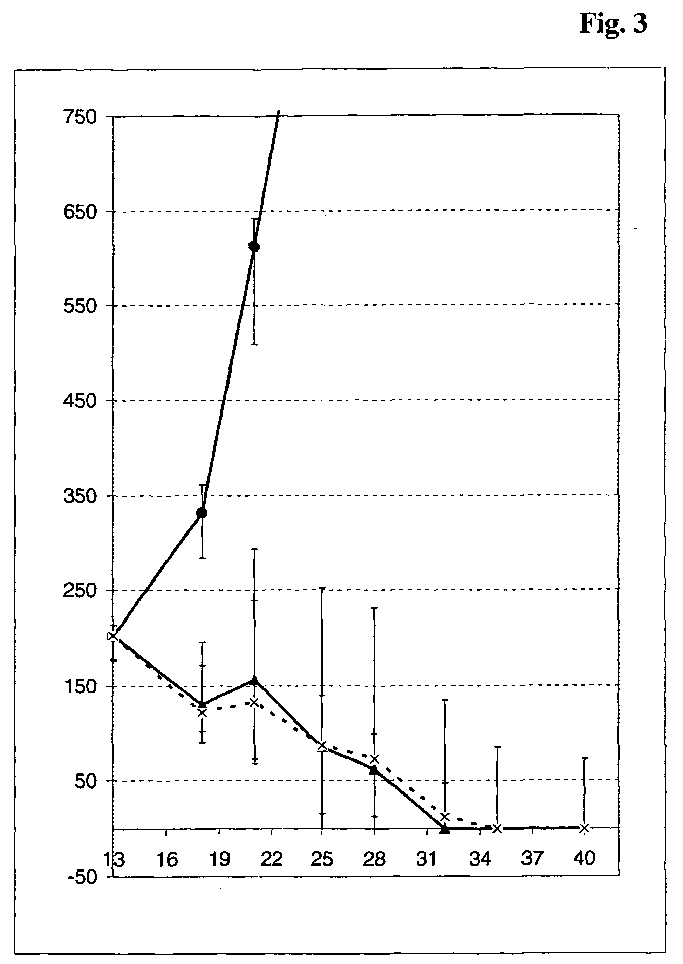

**Figure 3**    Antitumor activity of treatment of two type II anti-CD20 antibodies on the Z138 human Non-Hodgkin-Lymphoma (NHL). Both antibodies are humanized B-Ly1 anti-CD20 antibodies; 1) B-HH6-B-KV1 glycoengineered (GE) and 2) B-HH6-B-KV1 wildtype (wt, non-glycoengineered). Mean values of tumor volume [mm$^3$] plotted on the y-axis; number of days after injection of tumor cells plotted on the x-axis. Legend: A)Vehicle (*circles*), B) humanized B-ly1 GE (B-HH6-B-KV1 GE) 30 mg/kg once weekly (*triangles*) and C) humanized B-ty1 wt (B-HH6-B-KV1 wt) 30 mg/kg once weekly (crosses)

## Experimental Procedures

## Example 1

## Antitumor activity of combined treatment of a type I anti-CD20 antibody (rituximab) with a type II anti-CD20 antibody (B-HH6-B-KV1 GE)

### Test agents

Type I anti-CD20 antibody rituximab was provided as stock solution (c=10 mg/ml) from Hoffmann La Roche, Basel, Switzerland. Buffer contains polysorbate 80, Sodiumchloride and Sodiumcitrat.

Type II anti-CD20 antibody B-HH6-B-KV1 GE (= humanized B-Ly1, glycoengineered B-HH6-B-KV1, see WO 2005/044859 and WO 2007/031875)) was provided as stock solution (c=9.4 mg/kg) from GlycArt, Schlieren, Switzerland. Antibody buffer included histidine, trehalose and polysorbate 20

Both solutions were diluted appropriately in PBS from stock for prior injections.

### Cell lines and culture conditions

OCI-Ly18 human Non-Hodgkin-Lymphoma (NHL) cells (Chang, H., et al, Leuk Lymphoma. 1992 Sep;B(1-2): 129-36) (diffuse large cell lymphoma-DLCL) was used. Tumor cell line was routinely cultured in INDM medium (PAA, Laboratories, Austria) supplemented with 20 % fetal bovine serum (PAA Laboratories, Austria) and 2 mM L-glutamine, 25 nM HEPES and 0.05 mM mercaptoethanol at 37 °C in a water-saturated atmosphere at 5 % $CO_2$. Passage 2 was used for transplantation.

### Animals

Female SCID beige mice; age 4-5 weeks at arrival (purchased from Bomholtgard, Ry, Denmark) were maintained under specific-pathogen-free condition with daily cycles of 12 h light /12 h darkness according to committed guidelines (GV-Solas; Felasa; TierschG). Experimental study protocol was reviewed and approved by local government. After arrival animals were maintained in the quarantine part of the animal facility for one week to get accustomed to new environment and for observation. Continuous health monitoring was carried out on regular basis. Diet food (Provimi Kliba 3337) and water (acidified pH 2.5-3) were provided ad libitum.

### Monitoring

Animals were controlled daily for clinical symptoms and detection of adverse effects. For monitoring throughout the experiment body weight of animals was documented two times weekly and tumor volume was measured by caliper after staging.

### Treatment of animals

Animal treatment started at day of randomisation, 24 days after cell transplantation. Humanized type II anti-CD20 antibody B-HH6-B-KV1 GE receiving groups and the corresponding vehicle group were treated i.v. q7d on study day 24, 31, 38, 45 and 52 at the indicated dosage of 30 mg/kg. Type I anti-CD20 antibody rituximab treatment as single agent and in combination with type II anti-CD20 antibody B-HH6-B-KV1 GE was performed on day 26, 33, 40, 47 and 54

**Tumor growth inhibition study in vivo**

Tumor bearing animals receiving vehicle control had to be excluded 10 days after treatment initiation due to tumor burden. Treatment of animals with weekly Rituximab at 30 mg/kg as single agent inhibited xenograft growth for 10 days (TGI 68%). Later on tumor xenografts progressed continuously despite further weekly Rituximab single agent injections. In contrast single agent therapy with B-HH6-B-KV1 GE (30 mg/kg) once weekly controlled OCI-Ly18 tumor growth (TGI 100%). Nevertheless, finally tumor xenografts started to progress under B-HH6-B-KV1 GE single agent administration. However, combination of Rituximab and B-HH6-B-KV1 GE , both at 30 mg/kg, was obviously superiorly efficacious. Xenograft tumors were controlled and in contrast to each single agent antibody arm tumor stasis maintained over time.

## Example 2

### Determination of the ratio of the binding capacities to CD20 on Raji cells (ATCC-No. CCL-86) of type II anti-CD20 antibody compared to rituximab

Raji cells (ATCC-No. CCL-86) were maintained in culture in RPMI-1640 medium (PanBiotech GmbH, Cat.-No. PO4-18500) containing 10% FCS (Gibco, Cat.-No.10500-064). The type II anti-CD20 antibody B-HH6-B-KV1 (humanized B-Ly1 antibody) and rituximab were labeled using Cy5 Mono NHS ester (Amersham GE Healthcare, Catalogue No. PA15101) according to the manufacturer's instructions. Cy5-conjugated rituximab had a labeling ratio of 2.0 molecules Cy5 per antibody. Cy5-conjugated B-HH6-B-KV1 had a labeling ratio of 2.2 molecules Cy5 per antibody. In order to determine and compare the binding capacities and mode of both antibodies, binding curves ( by titration of Cy5-conjugated Rituximab and Cy5-conjugated B-HH6-B-KV1) were generated by direct immunofluorescence using the Burkitt's lymphoma cell line Raji (ATCC-No. CCL-86). Mean fluorescence intensities (MFI) were analyzed as EC50 (50% of maximal intensity) for Cy5-conjugated Rituximab and Cy5-conjugated B-HH6-B-KV1, respectively. 5* 105 cells per sample were stained for 30 min at 4 °C. Afterwards, cells were washed in culture medium. Propidium iodide (PI) staining was used to exclude dead cells. Measurements were performed using the FACSArray (Becton Dickinson), Propidium iodide (PI) was measured at Far Red A and Cy5 at Red-A. Figure 2 shows Mean Fluorescence Intensity (MFI) for binding at EC50 (50% of maximal intensity) of Cy5-labeled B-HH6-B-KV1 (black bar) and Cy5-labeled rituximab (white bar).

Then the ratio of the binding capacities to CD20 on Raji cells (ATCC-No. CCL-86) is calculated according to the following formula:

$$\text{Ratio of the binding capacities to CD20 on Raji cells (ATCC-No. CCL-86)} =$$

$$\frac{\text{MFI(Cy5-anti-CD20 antibody)}}{\text{MFI(Cy5-rituximab)}} \times \frac{\text{Cy5 labeling ratio(Cy5-rituximab)}}{\text{Cy5 labeling ratio(Cy5-anti-CD20 antibody)}}$$

$$= \frac{\text{MFI(B-HH6-B-KV1)}}{\text{MFI(Cy5-rituximab)}} \times \frac{\text{Cy5 labeling ratio(Cy5-rituximab)}}{\text{Cy5 labeling ratio(B-HH6-B-KV1)}}$$

$$= \frac{207}{433} \times \frac{2.2}{2.0} = 0.44$$

Thus B-HH6-B-KV as a typical type II anti-CD20 antibody shows reduces binding capacity compared to rituximab.

## Example 3

### Similar antitumor activity of glycoengineered (GE) and non-glycoengineered (wildtype, wt) anti-CD20 antibody (B-HH6-B-KV1 GE and wt) against Z138 MCL xenografts in SCID beige mice

### Test agents

Type II anti-CD20 antibody B-HH6-B-KV1 (glycoengineered (GE) and wildtype (wt)) were provided as stock solution (c=9.4 mg/ml and 12.5 mg/ml) from GlycArt, Schlieren, Switzerland. Antibody buffer included histidine, trehalose and polysorbate 20.
Both solutions were diluted appropriately in PBS from stock for prior injections.

Cell lines and culture conditions

Z138 human B-Cell Non-Hodgkin-lymphoma (NHL) cells were originally obtained from Glycart (Mantle cell lymphoma-MCL). Tumor cell line was routinely cultured in DMEM medium (PAA, Laboratories, Austria) supplemented with 10 % fetal bovine serum (PAA Laboratories, Austria) and 2 mM L-glutamine at 37 °C in a water-saturated atmosphere at 5 % $CO_2$. Passage 2 was used for transplantation.

### Animals

Female SCID beige mice; age 4-5 weeks at arrival (purchased from Bomholtgard, Ry, Denmark) were maintained under specific-pathogen-free condition with daily cycles of 12 h light /12 h darkness according to committed guidelines (GV-Solas; Felasa; TierschG). Experimental study protocol was reviewed and approved by local government. After arrival animals were maintained in the quarantine part of the animal facility for one week to get accustomed to new environment and for observation. Continuous health monitoring was carried out on regular basis. Diet food (Provimi Kliba 3337) and water (acidified pH 2.5-3) were provided ad libitum.

Monitoring

Animals were controlled daily for clinical symptoms and detection of adverse effects. For monitoring throughout the experiment body weight of animals was documented two times weekly and tumor volume was measured by caliper beginning at staging.

### Treatment of animals

Animal treatment started at day of randomisation, 14 days after s.c. cell transplantation. Humanized anti CD20 antibody (B-HH6-B-KV1 GE and wt) receiving groups and the corresponding vehicle group were treated i.v. q7d on study day 14, 20, 27 and 34 at the indicated dosage of 10 mg/kg.

### Tumor growth inhibition study in vivo

Tumor bearing animals receiving vehicle control had to be excluded 19 days after treatment initiation due to tumor burden. Treatment of animals with weekly B-HH6-B-KV1 as wt or glycoengineered (B-HH6-B-KV1 GE and wt) at 10 mg/kg inhibited xenograft outgrowth shortly after start of treatment. At time of control termination all antibody tumors regressed and later most of Z138 tumor xenografts showed complete remission. No significant differences were observed between wt and glycoengineered versions of anti CD20 antibody B-HH6-B-KV1 in this xenograft model. This was not unlikely since mice do not express the correct Fc receptor on their NK cells and furthermore SCID beige mice are thought to be incompetent for NK-mediated ADCC due to severe triple immunodeficiency. Therefore s.c. xenografts models in SCID beige mice are not appropriate for mimicking human ADCC mediated effect with glycoengineered modified antibodies.

**Claims**

1. Use of a type I anti-CD20 antibody for the manufacture of a medicament for the treatment of a CD20 expressing cancer, **characterized in that** said type I anti-CD20 antibody is co-administered with a type II anti-CD20 antibody, **characterized in that** said type I anti-CD20 antibody is rituximab, said type II anti-CD20 antibody is a humanized B-Ly1 antibody, at least 40% or more of the oligosaccharides of the Fc region of said type II anti-CD20 antibody are non-fucosylated and said CD20 expressing cancer is a B-Cell Non-Hodgkin's lymphoma (NHL), and wherein said humanized B-Ly1 antibody comprises a variable region of the heavy chain (VH) of SEQ ID No.7 and a variable region of the light chain (VL) of SEQ ID No. 20.

2. Use according to claim 1, **characterized in that** said type II anti-CD20 antibody has increased antibody dependent cellular cytotoxicity (ADCC).

3. A type I anti-CD20 antibody for use in the treatment of a CD20 expressing cancer **characterized in that** said type I anti-CD20 antibody is co-administered with a type II anti-CD20 antibody **characterized in that** said type I anti-CD20 antibody is rituximab, said type II anti-CD20 antibody is a humanized B-Ly1 antibody, at least 40% or more of the oligosaccharides of the Fc region of said type II anti-CD20 antibody are non-fucosylated and said CD20 expressing cancer is a B-Cell Non-Hodgkin's lymphoma (NHL), and wherein said humanized B-Ly1 antibody comprises a variable region of the heavy chain (VH) of SEQ ID No.7 and a variable region of the light chain (VL) of SEQ ID No. 20.

4. The type I anti-CD20 antibody according to claim 3 for use in the treatment of a CD20 expressing cancer according to claim 3, **characterized in that** said type II anti-CD20 antibody has increased antibody dependent cellular cytotoxicity (ADCC).

**Patentansprüche**

1. Verwendung eines Typ-I-anti-CD20-Antikörpers für die Herstellung eines Medikaments zur Behandlung von CD20 exprimierendem Krebs, **dadurch gekennzeichnet, dass** der Typ-I-anti-CD20-Antikörper gleichzeitig mit einem Typ-II-anti-CD20-Antikörper verabreicht wird, **dadurch gekennzeichnet, dass** der Typ-I-anti-CD20-Antikörper Rituximab ist, der Typ-II-anti-CD20-Antikörper ein humanisierter B-Ly1-Antikörper ist, mindestens 40% oder mehr der Oligosaccharide der Fc-Region des Typ-II-anti-CD20-Antikörpers nicht fucosyliert sind und der CD20 exprimierende Krebs ein B-Zell-Non-Hodgkin's Lymphom (NHL) ist, und wobei der humanisierte B-Ly1-Antikörper eine variable Region der schweren Kette (VH) mit der SEQ ID NO:7 und eine variable Region der leichten Kette (VL) mit der SEQ ID NO:20 umfasst.

2. Verwendung nach Anspruch 1, **dadurch gekennzeichnet, dass** der Typ-11-anti-CD20-Antikörper erhöhte Antikörper-abhängige zelluläre Cytotoxizität hat (ADCC).

3. Typ-I-anti-CD20-Antikörper zur Verwendung in der Behandlung von CD20-exprimierendem Krebs, **dadurch gekennzeichnet, dass** der Typ-I-anti-CD20-Antikörper gleichzeitig mit einem Typ-II-anti-CD20-Antikörper verabreicht wird, **dadurch gekennzeichnet, dass** der Typ-I-anti-CD20-Antikörper Rituximab ist, der Typ-II-anti-CD20-Antikörper humanisierter B-Ly1-Antikörper ist, mindestens 40% oder mehr der Oligosaccharide der Fc-Region des Typ-II-anti-CD20-Antikörpers nicht fucosyliert sind und der CD20 exprimierende Krebs ein B-Zell-Non-Hodgkin's Lymphom (NHL) ist, und wobei der humanisierte B-Ly1-Antikörper eine variable Region der schweren Kette (VH) mit der SEQ ID NO:7 und eine variable Region der leichten Kette (VL) mit der SEQ ID NO:20 umfasst.

4. Typ-I-anti-CD20-Antikörper nach Anspruch 3 zur Verwendung in der Behandlung von CD20-exprimierendem Krebs nach Anspruch 3, **dadurch gekennzeichnet, dass** der Typ-II-anti-CD20-Antikörper erhöhte Antikörper-abhängige zelluläre Cytotoxizität hat (ADCC).

**Revendications**

1. Utilisation d'un anticorps anti-CD20 de type I pour la préparation d'un médicament destiné à traiter un cancer exprimant CD20, **caractérisée en ce que** ledit anticorps anti-CD20 de type 1 est co-administré avec un anticorps anti-CD20 de type II, **caractérisée en ce que** ledit anticorps anti-CD20 de type I est le rituximab, ledit anticorps anti-CD20 de type II est un anticorps B-Ly1 humanisé, au moins 40 % ou plus des oligosaccharides de la région Fc dudit anticorps anti-CD20 de type II sont non fucosylés et ledit cancer exprimant CD20 est un lymphome B non hodgkinien (NHL), et dans laquelle ledit anticorps B-Ly1 humanisé comprend une région variable de la chaîne lourde (VH) de SEQ ID No. 7 et une région variable de la chaîne légère (VL) de SEQ ID No. 20.

2. Utilisation selon la revendication 1, **caractérisée en ce que** ledit anticorps anti-CD20 de type II présente une cytotoxicité cellulaire dépendante des anticorps (ADCC) accrue.

3. Anticorps anti-CD20 de type 1, pour une utilisation dans le traitement d'un cancer exprimant CD20, **caractérisé en ce que** ledit anticorps anti-CD20 de type 1 est co-administré avec un anticorps anti-CD20 de type II, **caractérisé en ce que** ledit anticorps anti-CD20 de type I est le rituximab, ledit anticorps anti-CD20 de type II est un anticorps B-Ly1 humanisé, au moins 40 % ou plus des oligosaccharides de la région Fc dudit anticorps anti-CD20 de type II sont non fucosylés et ledit cancer exprimant CD20 est un lymphome B non hodgkinien (NHL), et dans lequel ledit anticorps B-Ly1 humanisé comprend une région variable de la chaîne lourde (VH) de SEQ ID No. 7 et une région variable de la chaîne légère (VL) de SEQ ID No. 20.

4. Anticorps anti-CD20 de type I selon la revendication 3, pour une utilisation dans le traitement d'un cancer exprimant CD20 selon la revendication 3, **caractérisé en ce que** ledit anticorps anti-CD20 de type II présente une cytotoxicité cellulaire dépendante des anticorps (ADCC) accrue.

**Fig. 1**

# Fig. 2

**Fig. 3**

**REFERENCES CITED IN THE DESCRIPTION**

**Patent documents cited in the description**

- WO 2004035607 A
- US 5202238 A
- US 5204244 A
- WO 2005103081 A
- WO 2004056312 A
- WO 2005044859 A
- US 6602684 B
- US 5736137 A, Andersen
- WO 2007031875 A
- WO 2004065540 A
- WO 99154342 A
- WO 9960023 A
- US 3773919 A
- EP 07017337 A

**Non-patent literature cited in the description**

- **VALENTINE, M.A. et al.** *J. Biol. Chem.,* 1989, vol. 264 (19), 11282-11287
- **EINFIELD, D.A. et al.** *EMBO J.,* 1988, vol. 7 (3), 711-717
- **ANDERSON, K.C. et al.** *Blood,* 1984, vol. 63 (6), 1424-1433
- **TEDDER, T.F. et al.** *J, Immunol.,* 1985, vol. 135 (2), 973-979
- **KOMAROMY, M. et al.** *NAR,* 1983, vol. 11, 6775-6785
- **TEDDER et al.** *Eur. J. Immunol.,* 1986, vol. 16, 881-887
- **TEDDER, T.F. et al.** *J. Cell. Biochem.,* 1990, vol. 14D, 195
- **CRAGG, M.S. et al.** *Blood,* 2004, vol. 103, 2738-2743
- **CRAGG, M.S. et al.** *Blood,* 2003, vol. 101, 1045-1052
- **MORRISON, S.L. et al.** *Proc. Natl. Acad Sci. USA,* 1984, vol. 81, 6851-6855
- **RIECHMANN, L. et al.** *Nature,* 1988, vol. 332, 323-327
- **NEUBERGER, M.S. et al.** *Nature,* 1985, vol. 314, 268-270
- **VAN DIJK, M.A. ; VAN DE WINKEL, J.G.** *Curr. Opin. in Chemical Biology,* 2001, vol. 5, 368-374
- **KELLERMANN, S. A et al.** *Curr Opin Biotechnol.,* 2002, vol. 13, 593-597
- **KABAT, E.A. et al.** Sequences of Proteins of Immunological Interest. Public Health Service, National Institutes of Health, 1991
- **CRAGG, M.S et al.** *Blood,* 2004, vol. 103, 2738-2743
- **CRAGG, M.S et al.** *Blood,* 2003, vol. 101, 1045-1052
- **UMANA, P. et al.** *Nature Biotechnol.,* 1999, vol. 17, 176-180
- **REIFF, M.E.** *Blood,* 1994, vol. 83 (2), 435-445
- **POPPEMA, S. ; VISSER, L.** *Biotest Bulletin,* 1987, vol. 3, 131-139
- **JENKINS, N. et al.** *Nature Biotechnol.,* 1996, vol. 14, 975-81
- **CUMMING, D.A. et al.** *Glycobiology,* 1991, vol. 1, 115-30
- **WRIGHT, A. ; MORRISON, S. L.** *Trends Biotech.,* 1997, vol. 15, 26-32
- **LIFELY, M. R. et al.** *Glycobiology,* 1995, vol. 5 (8), 813-22
- **LIFELY, M. R. et al.** *Glycobiology,* 1995, vol. 5, 813-822
- **JEFFERIS, R. et al.** *Immunol. Rev,* 1998, vol. 163, 59-76
- **WRIGHT, A. ; MORRISON, S. L.** *Trends Biotechnol.,* 1997, vol. 15, 26-32
- Remington's Pharmaceutical Sciences. 1980
- **CHANG, H. et al.** *Leuk Lymphoma,* September 1992, vol. B (1-2), 129-36